# EUROPEAN PATENT APPLICATION

(11) **EP 1 561 819 A1**
(43) Date of publication of application: **10.08.2005**
(21) Application number: 03758769.8
(22) Date of filing: 22.10.2003
(51) Int. Cl.: C12N 15/86, C12N 5/10, C12N 7/01

(54) **METHOD OF TRANSFERRING GENE INTO T CELLS**

(30) Priority: 24.10.2002 JP 2002310053
(71) Applicant: Dnavec Research Inc., Tsukuba-shi, Ibaraki 305-0856 (JP)
(72) Inventor: OKANO, Shinji, Fukuoka-shi, Fukuoka 812-0041 (JP); YONEMITSU, Yoshikazu, Fukuoka-shi, Fukuoka 813-0043 (JP); SUEISHI, Katsuo, Fukuoka-shi, Fukuoka 815-0073 (JP); HASEGAWA, Mamoru, c/o DNAVEC RESEARCH INC., Tsukuba-shi, Ibaraki 305-0856 (JP)
(74) Representative: VOSSIUS & PARTNER
(86) International application number: PCT/JP2003/013476
(87) International publication number: WO 2004/038029

(57) **Abstract**

The present invention provides methods of transducing a gene into activated T cells comprising the step of contacting a paramyxovirus vector with activated T cells. This invention also provides a method of preparing T cells transduced with a foreign gene comprising the step of contacting a paramyxovirus vector with activated T cells. This invention also provides T cells transduced with a foreign gene prepared by this method. The present invention enables efficient gene transduction specific to activated T cells, and is expected to be applied to immunological modification strategies using T cell-directed gene delivery.

## Description

### Technical Field

The present invention relates to methods of transducing genes into T cells.

### Background Art

Genetic modification of hematopoietic cells is an attractive strategy for treating autoimmune diseases, immunodeficiencies, as well as tumors via the activation of antitumor immunity. Among the various blood cells, T lymphocytes have been a target for gene delivery since the early stage of ADA-SCID (severe combined immunodeficiency disease due to adenosine deaminase deficiency) gene therapy (Blaese, R.M. *et al.,* Science, 1995, 270: 475-480; Altenschmidt, U. *et al.*, J. Mol. Med., 1997, 75: 259-266; Misaki, Y. *et al.,* Mol. Ther., 2001, 3: 24-27). However, T cells are relatively resistant to gene delivery using presently available vectors such as retroviruses, which has currently become an obstacle for gene delivery.

Considering clinical settings in treating autoimmune diseases, rejection following organ allo-transplantation, tumors, or such, subsets of activated T lymphocytes are evidently ideal targets for genetic modification (Altenschmidt, U. *et al.,* J. Mol. Med., 1997, 75: 259-266; Hege, K.M. and Roberts, M.R., Curr. Opin. Biotechnol., 1996, 7: 629-634; Tuohy, V.K. *et al.,* J. Neuroimmunol., 2000, 107: 226-232). Early clinical reports on gene marking proved that tumor infiltrating lymphocytes (TILs), which are activated by tumor antigens, migrate to tumors in tumor-bearing individuals, suggesting that TILs are an ideal carrier vehicle for delivering therapeutic genes into tumors (Rosenberg, S.A. *et al.,* N. Engl. J. Med., 1990, 323: 570-578). Similar characteristics are expected of activated T lymphocytes in autoimmunity and organ transplantation. However, very little of such research has been performed in the past 10 years presumably because of the low efficiency of gene delivery into activated T cells by the presently available vectors.

Recently, a newly discovered vehicle for gene delivery into T lymphocytes, namely the human immunodeficiency virus (HIV)-based lentiviral vector, has received considerable attention because of its CD4 lymphocyte-directed HIV tropism. As a result of efforts made in the recent years, efficiency of HIV gene delivery into activated T cells has been dramatically improved through the construction of a central DNA flap, which promotes the import of vector genome into the nucleus and its integration into chromosomes on an average of 51% versus the 15% achieved by using a conventional HIV vector (Dardalhon, V. *et al.,* Gene Ther., 2001, 8: 190-198). However, in the case of lentiviral vectors based on pathogenic viruses including HIV in particular, potential safety concerns have delayed their clinical applications (Buchschacher, G.L. Jr. and Wong-Staal, F., Blood 2000, 95: 2499-2504). Continuous efforts are unquestionably necessary for developing safer alternatives that allow more efficient gene delivery into T cells.

### Disclosure of the Invention

To search for vectors that can efficiently transduce genes into T cells, vectors were transferred into T cells under various conditions and gene transfer efficiency was measured. As a result, the present inventors discovered that paramyxovirus vectors have a high gene transduction efficiency towards antigen-activated T cells. The gene transduction was specific to antigen-activated T cells, that is, the vectors' gene transduction efficiency towards activated T cells was remarkably higher compared to naive T cells. Paramyxovirus vectors can be preferably used as vectors for gene transduction into antigen-activated T cells.

Although T lymphocyte-directed gene therapy presents a possibility for treating various immunological diseases, the low efficiency of gene transduction despite the necessity to perform complicated procedures has been a major constraint in T cell-directed gene therapy thus far. The present invention has proved that paramyxovirus vectors can be made to specifically express a foreign gene in activated T cells using a very simple procedure, and therefore has overcome the above-described problems of T cell-directed gene therapy. As a result of the present invention, gene transduction specific to activated T cells has become possible. Therefore, the present invention is expected to be applied in immunological modification strategies using T cell-directed gene delivery in immune diseases.

That is, the present invention relates to methods of transducing genes into T cells, more specifically to:
(1) a method for transducing a gene into T cells, wherein said method comprises the step of contacting a paramyxovirus vector carrying the gene with activated T cells;
(2) the method according to (1), wherein the paramyxovirus vector is a Sendai virus vector;
(3) a method of preparing T cells transduced with a foreign gene, wherein said method comprises the step of contacting a paramyxovirus vector carrying said gene with activated T cells;
(4) the method according to (3), wherein the paramyxovirus vector is a Sendai virus vector;
(5) a T cell transduced with a foreign gene prepared by the method according to (3) or (4);
(6) a paramyxovirus vector to be used in gene transduction into activated T cells; and,
(7) the vector according to (6), wherein the paramyxovirus vector is a Sendai virus vector.

The present invention provides a method for transducing a gene into T cells using a paramyxovirus vector, comprising the step of contacting a paramyxovirus vector carrying a gene to be transduced with activated T cells. The present inventors discovered that this paramyxovirus vector is capable of transducing a gene into activated T cells with an extremely high efficiency. The low gene transduction efficiency of the paramyxovirus vector towards naive T cells indicates that gene transduction by this vector is specific to antigen-activated T cells. Therefore, the method of this invention can be preferably utilized to selectively transduce genes into activated T cells. T cells are important as targets for controlling the immune system in treating cancer and other diseases, and the method of this invention can be suitably used in gene therapy for these diseases. Gene transduction can be performed in any desired physiological aqueous solutions such as culture media, physiological saline, blood, and body fluids.

Further, this invention provides a method for transducing a desired gene into T cells, which comprises the steps of: (a) activating T cells, and (b) contacting the activated T cells with a paramyxovirus vector carrying a desired gene. This method is also included in the present invention's methods of transducing a gene into T cells. T cells can be activated by antigen stimulation. The step of activating T cells enables efficient gene transduction by paramyxovirus vectors. T cell activation may be performed in the presence of a paramyxovirus vector or prior to contacting a paramyxovirus vector with these T cells.

One important advantage of T cell-targeted gene delivery via paramyxovirus vectors is that it allows highly efficient gene transduction with a simple technique. As previously reported, gene delivery into T cells using retroviruses and lentiviruses requires that the lymphocytes be concentrated by centrifugation for optimum gene delivery, and also requires the use of toxic drugs such as polybrene (Bunnell, B.A. *et al.,* Proc. Natl. Acad. Sci. U S A, 1995, 92: 7739-7743; Chuck, A.S., Hum. Gene Ther., 1996, 7: 743-750; Chinnasamy, D. *et al.,* Blood 2000, 96: 1309-1316; Fehse, B. *et al.,* Br. J. Haematol., 1998, 102: 566-574). On the other hand, paramyxoviral solutions could achieve superior gene transduction, requiring only a simple addition without the help of any special drugs. Further, similarly to the typical results observed with nasal mucosa (Yonemitsu, Y. *et al.,* Nat. Biotechnol., 2000, 18: 970-973), vasculature (Masaki, I. *et al.,* FASEB J., 2001, 15: 1294-1296), retinal tissue (Ikeda, Y. *et al.,* Exp. Eye Res., 2002, 75: 39-48), and such, optimum gene delivery into activated T cells via Sendai virus vector (SeV) could be performed by a relatively short exposure of T cells (less than 30 min, data not shown). From a clinical point of view, these characteristics of paramyxovirus vector-mediated gene delivery can simplify the *ex vivo* genetic modification of T lymphocytes in this invention and minimize loss of cell viability in the process.

The present inventors discovered that *in vitro,* the lower the cell density of human lymphocytes, the lower the ratio of gene-transduced cells, and observed the same result with murine cells. Therefore, the cell density for gene transduction in the presesnt invention is preferably relatively high. A cell density in the range of approximately, for example, 1x 10⁶ /ml to 4x 10⁶/ml, preferably 4x 10⁶/ml to 8x 10⁶ /ml, more preferably 8x 10⁶/ml to 1x 10⁷/ml, is appropriate.

The vector is administered at an MOI (multiplicity of infection) in the range of preferably 1 to 500, more preferably 2 to 300, and even more preferably 3 to 200. Short contact of the vector with T cells, for example, 1 minute or longer, preferably 3 minutes or longer, 5 minutes or longer, or 10 minutes or longer, is sufficient. Alternatively, the contact time may be 20 minutes or longer, for example, approximately 1 to 60 minutes, more specifically 5 to 30 minutes. Needless to say, the contact can be longer than this, such as several days or longer.

Recently, sufficient numbers of T cell clones can be easily prepared using several refined and efficient techniques, including the universal artificial antigen-presenting cell (APC) system which is capable of stimulating T cells with anti-CD3 and anti-CD28, as well as 4-1BB ligand using immunological synapses (Maus, M.V. *et al.,* Nat. Biotechnol., 2002, 20: 143-148). By combining such techniques, the vector system derived from SeV would have a significant therapeutic potential in the clinical applications of T cell-directed gene therapy against various immunological disorders.

This invention also provides a method of selectively transducing a gene into activated T cells, comprising the step of allowing a paramyxovirus vector carrying a gene to coexist with a cell population containing activated and naive T cells. "Selectively transducing a gene into activated T cells" means that an activated T cell has been significantly transduced compared to naive T cells. For example, this invention provides a method comprising the step of adding a parmyxovirus vector carrying the gene to a cell population containing activated and naive T cells. Since the paramyxovirus vector transduces a gene into activated T cells preferentially over naive T cells, this method enables selective gene transduction into activated T cells. Alternatively, a vector can be selectively transduced into activated T cells by allowing T cells to coexist with the vector and then activating these T cells through treatments. These methods are also included in the method of transducing a gene into T cells in this invention.

T cells, also referred to as T lymphocytes, express T cell receptors which recognize the antigen peptide complex presented by the major histocompatibility complex (MHC). Mainly, T cells differentiate from bone marrow stem cells, undergo positive selection (selection of T cell repertoires that recognize self MHC) and negative selection (elimination of T cell repertoires that recognize self antigens) in the thymus, and appear as mature naive T cells in the peripheral blood and lymphoid tissues. T cells are major lymphocytes that recognize peptides derived from protein antigens, tumor antigens, allo-antigens, pathogens, and others and generate antigen-specific immune response (adaptive immunity) in individuals. T cells assist in the production of antibodies against these peptides (humoral immunity) or induce cellular immunity by becoming armed T cells themselves.

Activated T cells refer to T lymphocytes that are in such a state that proliferation/differentiation is induced when stimulated by antigens, mitogens, etc. In brief, activated T cells refer to T cells that undergo DNA synthesis, cell division, and proliferatiion/differentiation as a result of intracellular tyrosine kinase activation by T cell receptor binding or direct enzyme activation, which is followed by acceleration of inositol phopholipid metabolism and increase in intracellular calcium concentration, production of interleukin (IL)-2 and expression of IL-2 receptor, as well as generation of additional cellular signals. Various cytokines are produced from various types of differentiated T cells depending on the biological environment at the time of T cell activation.

Further, activated T cells in this invention are preferably T cells activated by antigens. Gene transduction via Sendai virus vectors is selective for antigen-activated T cells. Transduction efficiency is low in the case of antigen-nonspecific T cells, which are bystander-activated from specific T cells that have responded to antigens *ex vivo.* Therefore, vector-mediated gene transduction efficiency can be dramatically improved by activating T cells with antigens, or by performing an equivalent activation.

Antigen-activated T cells refer to T cells that have receptors with an appropriate affinity for the above-described complexes of the antigen-presenting cells' MHC with peptides or such derived from a specific antigen, and that transduce activation signals via the binding of the receptors to the complexes. Preferably, antigen-activated T cells refer to T cells activated by signal transduction via appropriate co-receptors such as CD28 and 4-1BB. Preferably, antigen-activated T cells are capable of proliferation, blast formation, production of various cytokines such as IL-2, IL-4, and IFN-γ, expression of cytotoxic molecules such as Fas Ligand and perforin, activation of antigen (such as CD40-ligand)-presenting cells and/or B cells, and so on. Antigen-specific T cells activate antigen-presenting cells and/or B cells and stimulate antibody production in lymph nodes and such, under the presence of MHC and antigen-presenting cells which presents the peptide. In localized peripheral tissues, antigen-specific T cells have the major function of excluding non-self proteins, non-self cells, and pathogens from the living body through actions such as induction of cytotoxicity and inflammation due to generated cytokines and cytotoxic molecules.

Activated T cells can be prepared by fractionation. For example, activated T cells can be separated from naive T cells based on the characteristic that human T cells alter the expression pattern of CD antigens upon activation. A specific method involves collecting T cells through negative selection, and sorting with an antibody against CD45RO exposed on activated T cells by using the magnetic bead separation method or flow cytometry. CD45RA+ and CD62L+ double-positive T cells are naive T cells, and the rest is considered either activated or memory T cells. Therefore, antibodies against both CD45RA and CD62L can be preferably used to sort and obtain fractions of activated T cells or naive T cells, by using the magnetic bead separation method or flow cytometry. Antibodies used in fractionation can be used in all methods that employ combinations of known markers associated with T cell activation. Further, the method of fractionating activated T cells into populations with specialized functions, such as those with chemokine receptors or cytokine receptors, is also included in this invention. Fractionation methods also include known methods such as those that use relative densities.

Activated T cells can also be prepared by activating naive T cells through antigenic stimulation. For example, naive T cells can be activated by culturing them in a plate having immobilied anti-CD3 antibody (10 µg/ml) and anti-CD28 antibody (10 µg/ml) at the concentrations described below, preferably with the simultaneous addition of mature dendritic cells differentiated from peripheral blood monocytes. Further, as described in the section of activation by tumor antigen, naive T cells can also be activated in cultures supplied with dendritic cells and peptides or protein antigens.

For example, when a human alloantigen is used, antigen-activated T cells can be prepared by: collecting peripheral blood samples from a donor and a recipient; separating lymphocytes from each sample using a peripheral blood lymphocyte separation solution; adjusting the lymphocyte suspensions to a concentration of 1x10⁷ cells/ml; pipetting the recipeint cell suspension and 30 Gy-irradiated donor-derived cell suspension (500 µl each) into each well of a 24-well plate; and culturing in the presence of human IL-2 (5 to 100 U/ml) for about 7 days. Subculturs can be re-stimulated every 7 days the with irradiated donor lymphocytes. An antigen-specific T cell line is preferably one that has undergone antigen stimulation at least three times (including the first one). Alternatively, T cells which have been stimulated to proliferate after a second antigen stimulation using, for example, beads or cells having immobilized anti-CD3 and anti-CD28 antibodies, are also included in the antigen-activated T cells.

T cells activated by tumor antigens and such can be obtained by subjecting tumor cells to quick freeze-thawing (four cycles or more), adding the cell lysates to dendritic cells differentiated from peripheral blood, using these cells as antigen-presenting cells following exposure to radiation of 20 to 30 Gy, co-culturing these antigen-presenting cells and T cells separated from peripheral blood, in the presence of IL-2 (5 to 100 U/ml) alone or with additional optimal cytokines such as IL-7 for 7 days, and re-stimulating thrice every 7 days (Fields, R.C. *et al.,* Proc. Natl. Acad. Aci. USA, 1998, 95: 9482-9487). Dendritic cells used herein include all of those obtained by known methods that use cytokines such as GM-CSF and IL-4 for the proliferation and differentiation of hematopoietic stem cells, such as peripheral blood monocytes, bone marrow, umbilical cord blood and mobilized peripheral blood.

Peripheral blood T cells may be separated using a T cell separation solution. When the effective peptide portion of an antigen is known, T cells may be obtained using methods for separating antigen-specific T cells via peptide complexes with Class I or Class II tetramers.

Apart from the above-described methods, when the specific antigen is known , activated T cells can also be prepared by activation methods using the antigen, or a peptide or protein derived from the antigen. Known methods for activating T cells such as the nonspecific activation method using lectins or the like can also be used to prepare activated T cells. Antigen-activated T cells in this invention also include these T cells thus obtained.

These activated T cells can be passaged by co-culturing with appropriate growth factors, cytokines and antigens, and antigen-presenting cells (including feeder cells, differentiated dendritic cells, or artificial APC), or with antigen presenting cells that do not present any antigens, and such. Other passage methods appropriate for the disorder that is treated may also be used. For example, in the case of transfer immunotherapy for infection immunity and such, T cells produce various cytokines in the midst of an antigen activation, and therefore, side effects such as fever are likely to occur upon entry of the T cells into a living body. Further, T cells used to transfer immunity must express their functions only at the time of infection. Therefore, there are a methods that prepare resting activated T cells (so-called memory T cells) by transducing a gene into antigen-activated T cells using a paramyxovirus vector, and then co-culturing with APC in the absence of the antigen.

In this invention, the paramyxovirus vector is a paramyxovirus-based virion with infectivity and a vehicle for transducing genes into cells. Herein, "infectivity" refers to the capability of a paramyxovirus vector to maintain cell-adhesion ability and transduce a gene carried by the vector to the inside of the cell to which the vector has adhered. In a preferable embodiment, the paramyxovirus vector of this invention has a foreign gene incorporated into its genomic RNA for expression. The paramyxovirus vector of this invention may have replication ability or may be a defective-type vector with no replication ability. "Having replication ability" means that when a viral vector infects a host cell, the virus is replicated in the cell to produce infectious virions.

"Recombinant virus" refers to a virus produced through a recombinant polynucleotide. "Recombinant polynucleotide" refers to a polynucleotide in which nucleotides are not linked at one or both ends as in the natural condition. Specifically, a recombinant polynucleotide is a polynucleotide in which the linkage of the polynucleotide chain has been artificially modified (cleaved and/or linked). Recombinant polynucleotides can be produced by using gene recombination methods known in the art in combination with polynucleotide synthesis, nuclease treatment, ligase treatment, etc. A recombinant virus can be produced by expressing a polynucleotide encoding a viral genome constructed through gene manipulation and reconstructing the virus. For example, recombinant paramyxovirus can be produced by reconstruction from cDNA (Y. Nagai, A. Kato, Microbiol. Immunol., 43, 613-624 (1999)).

In the present invention, "gene" refers to a genetic substance, a nucleic acid encoding a transcription unit. Genes may be RNAs or DNAs. In this invention, a nucleic acid encoding a protein is referred to as a gene of that protein. Further, a gene may not encode a protein. For example, a gene may encode a functional RNA, such as a ribozyme or antisense RNA. A gene may be a naturally-occurring or artificially designed sequence. Furthermore, in the present invention, "DNA" includes both single-stranded and double-stranded DNAs. Moreover, "encoding a protein" means that a polynucleotide comprises an ORF that encodes an amino acid sequence of the protein in a sense or antisense strand, so that the protein can be expressed under appropriate conditions.

In this invention, "paramyxovirus" refers to a virus belonging to Paramyxoviridae, or to derivatives thereof. Paramyxoviruses are a group of viruses with non-segmented negative strand RNA as their genome, and include Paramyxovirinae (including *Respirovirus* (also referred to as *Paramyxovirus*), *Rubulavirus,* and *Morbillivirus*), and Pneumovirinae virus (including *Pneumovirus* and *Metapneumovirus*)*.* Specific examples of *Paramyxovirus* applicable to the present invention are the Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3. More specifically, such examples include Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). A more preferred example is a virus selected from the group consisting of Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), and Nipah virus (Nipah). Viruses of this invention are preferably those belonging to Paramyxovirinae (including *Respirovirus, Rubulavirus,* and *Morbillivirus*) or derivatives thereof, and more preferably those belonging to the genus *Respirovirus* (also referred to as *Paramyxovirus*) or derivatives thereof. Examples of viruses of the genus *Respirovirus* applicable to this invention are human parainfluenza virus-1 (HPIV-1), human parainfluenza virus-3 (HPIV-3), bovine parainfluenza virus-3 (BPIV-3), Sendai virus (also referred to as murine parainfluenza virus-1), and simian parainfluenza virus-10 (SPIV-10). The most preferred paramyxovirus in this invention is the Sendai virus. These viruses may be derived from natural strains, wild strains, mutant strains, laboratory-passaged strains, artificially constructed strains, or the like.

Genes harbored on a paramyxovirus vector are situated in the antisense direction in the genomic RNA. Genomic RNA refers to RNA that has the function to form a ribonucleoprotein (RNP) with the viral proteins of a paramyxovirus. Genes contained in the genome are expressed by the RNP, genomic RNA is replicated, and daughter RNPs are formed. In general, the genome of a paramyxovirus is constituted so that the viral genes are situated in an antisense orientation between the 3'-leader region and 5'-trailer region. Between the ORFs of individual genes exists a transcription ending sequence (E sequence) - intervening sequence (I sequence) - transcription starting sequence (S sequence) that allows the RNA encoding each ORF to be transcribed as a separate cistron.

Genes encoding the viral proteins of a paramyxovirus include NP, P, M, F, HN, and L genes. "NP, P, M, F, HN, and L genes" refer to genes encoding nucleocapside-, phospho-, matrix-, fusion-, hemagglutinin-neuraminidase-, and large-proteins respectively. Genes in each virus belonging to Paramyxovirinae are commonly listed as follows. In general, NP gene is also listed as "N gene."
*Respirovirus*····NP···P/CV···M···F···HN····-····L
*Rubulavirus*·····NP···PV·····M··· F···HN···(SH)··L
*Morbillivirus*···NP···P/C/V···M···F···H·····-····L

For example, the database accession numbers for the nucleotide sequences of each of the Sendai virus genes are: M29343, M30202, M30203, M30204, M51331, M55565, M69046, and X17218 for NP gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Examples of viral genes encoded by other viruses are: CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for P gene; CDV, AF014953; DMV, Z47758; HPIV-1, M74081; HPIV-3, D00047; MV, ABO16162; RPV, X68311; SeV, AB005796; and Tupaia, AF079780 for C gene; CDV, M12669; DMV, Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for M gene; CDV, M21849; DMV, AJ224704; HPN-1, M22347; HPIV-2, M60182; HPIV-3, X05303; HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for F gene; and, CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, Z81358; PDV, Z36979; RPV, AF132934; SeV, U06433; and SV-5, S76876 for HN (H or G) gene. However, a number of strains are known for each virus, and genes exist that comprise sequences other than those cited above, due to strains differences.

The ORFs encoding these viral proteins and ORFs of the foreign genes are arranged in the antisense direction in the genomic RNAs, via the above-described E-I-S sequence. The ORF closest to the 3'-end of the genomic RNAs only requires an S sequence between the 3'-leader region and the ORF, and does not require an E or I sequence. Further, the ORF closest to the 5'-end of the genomic RNA only requires an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. In wild type paramyxoviruses, a typical RNA genome comprises a 3'-leader region, six ORFs encoding the N, P, M, F, HN, and L proteins in the antisense direction and in this order, and a 5'-trailer region on the other end. The viral gene orientation in the genomic RNAs of this invention are not restricted, but similarly to the wild type viruses, it is preferable that ORFs encoding the N, P, M, F, HN, and L proteins are arranged in this order, after the 3'-leader region, and before the 5'-trailer region. Certain types of paramyxoviruses have different viral genes, but even in such cases, it is preferable that each gene be arranged as in the wild type, as described above. In general, vectors maintaining the N, P, and L genes can autonomously express genes from the RNA genome in cells, replicating the genomic RNA. Furthermore, by the action of genes such as the F and HN genes, which encode envelope proteins, and the M gene, infectious virions are formed and released to the outside of cells. Thus, such vectors become viral vectors with replication ability. A foreign gene to be transduced into T cells may be inserted into a protein-noncoding region in this genome, as described below.

Further, a paramyxovirus vector of this invention may be deficient in any of the wild type paramyxovirus genes. For example, a paramyxovirus vector that does not comprise the M, F, or HN gene, or any combinations thereof, can be preferably used as a paramyxovirus vector of this invention. Such viral vectors can be reconstituted, for example, by externally supplying the products of the deficient genes. Similar to wild type viruses, the viral vectors thus prepared adhere to host cells and cause cell fusion, but they cannot form daughter virions that comprise the same infectivity as the original vector, because the vector genome introduced into cells is deficient in viral genes. Therefore, such vectors are useful as safe viral vectors that can only introduce genes once. Examples of genes that the genome may be deficient in are the F gene and/or HN gene. For example, viral vectors can be reconstituted by transfecting host cells with a plasmid expressing a recombinant paramyxovirus vector genome deficient in the F gene, along with an F protein expression vector and expression vectors for the NP, P, and L proteins (WO00/70055 and WO00/70070; Li, H.-O. *et al.,* J. Virol. 74(14) 6564-6569 (2000)). Viruses can also be produced, for example, by using host cells that have incorporated the F gene into their chromosomes. In these proteins, their amino acid sequences do not need to be the same as the viral sequences, and a mutant or homologous gene from another virus may be used as a substitute, as long as their activity in nucleic acid introduction is the same as, or greater than, that of the natural type.

Further, vectors that comprise an envelope protein other than that of the virus from which the vector genome was derived, may be prepared as viral vectors of this invention. For example, when reconstituting a virus, a viral vector comprising a desired envelope protein can be generated by expressing an envelope protein other than the envelope protein encoded by the basic viral genome. Such proteins are not particularly limited, and include the envelope proteins of other viruses, for example, the G protein of vesicular stomatitis virus (VSV-G). The viral vectors of this invention include pseudotype viral vectors comprising envelope proteins, such as VSV-G, derived from viruses other than the virus from which the genome was derived. If the viral vectors are designed such that these envelope proteins are not encoded in RNA genomes, the proteins will never be expressed after virion infection of the cells.

Furthermore, the viral vectors of this invention may be, for example, vectors comprising on the envelope surface thereof, proteins such as adhesion factors capable of adhering to specific cells, ligands, receptors, antibodies or fragments, or vectors comprising a chimeric protein with these proteins in the extracellular domain and polypeptides derived from the virus envelope in the intracellular domain. Thus, the T cell specificity of the vectors can be controlled. These proteins may be encoded in the viral genome, or supplied through the expression of genes not in the viral genome (for example, genes carried by other expression vectors, or genes in the host chromosomes) at the time of viral vector reconstitution.

Further, in the vectors of this invention, any viral gene comprised in the vector may be modified from the wild type gene in order to reduce the immunogenicity caused by viral proteins, or to enhance RNA transcriptional or replicational efficiency, for example. Specifically, for example, in a paramyxovirus vector, modifying at least one of the replication factors N, P, and L genes, is considered to enhance transcriptional or replicational function. Furthermore, although the HN protein, which is an envelope protein, comprises both hemagglutinin activity and neuraminidase activity, it is possible, for example, to improve viral stability in blood if the former activity can be attenuated, and infectivity can be controlled if the latter activity is modified. Further, it is also possible to control membrane fusion ability by modifying the F protein. For example, the epitopes of the F protein or HN protein, which can be cell surface antigenic molecules, can be analyzed, and using this, viral vectors with reduced antigenicity to these proteins can be prepared.

Furthermore, vectors of this invention may be deficient in accessory genes. For example, by knocking out the V gene, one of the SeV accessory genes, the pathogenicity of SeV toward hosts such as mice is remarkably reduced, without hindering gene expression and replication in cultured cells (Kato, A. *et al.,* 1997, J. Virol. 71: 7266-7272; Kato, A. *et al.,* 1997, EMBO J. 16: 578-587; Curran, J. *et al.,* WO01/04272, EP1067179). Such attenuated vectors are particularly useful as nontoxic viral vectors for *in vivo* or *ex vivo* gene transfer.

Paramyxoviruses are excellent gene transfer vectors. They do not have DNA phase and carry out transcription and replication only in the host cytoplasm, and consequently, chromosomal integration does not occur (Lamb, R.A. and Kolakofsky, D., Paramyxoviridae: The viruses and their replication. In: Fields BN, Knipe DM, Howley PM, (eds). Fields of Virology. Vol. 2. Lippincott - Raven Publishers: Philadelphia, 1996, pp. 1177-1204). Therefore, safety issues such as transformation and immortalization due to chromosomal abberation do not occur. This paramyxovirus characteristic contributes greatly to safety when it is used as a vector. For example, results on foreign gene expression show that even after multiple continuous passages of SeV, almost no nucletide mutation is observed. This suggests that the viral genome is highly stable and the inserted foreign genes are stably expressed over long periods of time (Yu, D. *et al.,* Genes Cells 2, 457-466 (1997)). Further, there are qualitative advantages associated with SeV not having a capsid structural protein, such as packaging flexibility and insert gene size, suggesting that paramyxovirus vectors may become a novel class of highly efficient vectors for human gene therapy. SeV vectors with replication ability are capable of introducing foreign genes of up to at least 4 kb in size, and can simultaneously express two or more kinds of genes by adding the transcriptional units.

Further, SeV is known to be pathogenic in rodents causing pneumonia, but is not pathogenic for human. This is also supported by a previous report that nasal administration of wild type SeV does not have severely harmful effects on non-human primates (Hurwitz, J.L. *et al.,* Vaccine 15: 533-540, 1997). These SeV characteristics suggest that SeV vectors can be applied therapeutically on humans, supporting the fact that SeV vectors are a promising choice of gene therapy that targets human T cells.

Viral vectors of this invention are capable of encoding foreign genes in their genomic RNA. A recombinant paramyxovirus vector harboring a foreign gene is obtained by inserting a foreign gene into an above-described paramyxovirus vector genome. The foreign gene can be any desired gene that needs to be expressed in a target T cell, and may be a gene that encodes a naturally-occurring protein, or protein modified from a naturally-occurring protein by deletion, substitution, or insertion of amino acid residues. The foreign gene can be inserted at any desired position in a protein-noncoding region of the virus genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region in genomic DNA. Further, in genomes deficient in the F or HN gene or the like, nucleic acids encoding the foreign genes can be inserted into those deficient regions. When introducing a foreign gene into a paramyxovirus, it is desirable to insert the gene such that the chain length of the polynucleotide to be inserted into the genome will be a multiple of six (Journal of Virology, Vol. 67, No. 8, 4822-4830, 1993). An E-I-S sequence should be arranged between the inserted foreign gene and the viral ORF. Two or more genes can be inserted in tandem via E-I-S sequences.

Expression levels of a foreign gene carried in a vector can be controlled using the type of transcriptional initiation sequence added upstream (to the 3'-side of the negative strand) of the gene (WO01/18223). The expression levels can also be controlled by the position at which the foreign gene is inserted in the genome: the nearer to the 3'-end of the negative strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high foreign gene expression level is thought to be advantageous, it is preferable to link the foreign gene to a highly efficient transcriptional initiation sequence, and to insert it near the 3'-end of the negative strand genome. Specifically, a foreign gene is inserted between the 3'-leader region and the viral protein ORF closest to the 3'-end. Alternatively, a foreign gene may be inserted between the ORFs of the viral gene closest to the 3'-end and the second closest viral gene. In wild type paramyxoviruses, the viral protein gene closest to the 3'-end of the genome is the N gene, and the second closest gene is the P gene. Alternatively, when a high level of expression of the introduced gene is undesirable, the gene expression level from the viral vector can be suppressed to obtain an appropriate effect, for example, by inserting the foreign gene at a site in the vector as close as possible to the 5'-side of the negative strand, or by selecting an inefficient transcriptional initiation sequence.

To prepare a vector of the present invention, a cDNA encoding a genomic RNA of a paramyxovirus is transcribed in mammalian cells, in the presence of viral proteins (i.e., N, P, and L proteins) essential for reconstitution of an RNP, which is a component of a paramyxovirus. Viral RNP can be reconstituted by producing either the negative strand genome (that is, the same antisense strand as the viral genome) or the positive strand (the sense strand encoding the viral proteins). Production of the positive strand is preferable for increased efficiency of vector reconstitution. The RNA terminals preferably reflect the terminals of the 3'-leader sequence and 5'-trailer sequence as accurately as possible, as in the natural viral genome. To accurately regulate the 5'-end of the transcript, for example, the RNA polymerase may be expressed within a cell using the recognition sequence of T7 RNA polymerase as a transcription initiation site. To regulate the 3'-end of the transcript, for example, a self-cleaving ribozyme can be encoded at the 3'-end of the transcript, allowing accurate cleavage of the 3'-end with this ribozyme (Hasan, M. K. *et al.,* J. Gen. Virol. 78: 2813-2820, 1997; Kato, A. *et al.,* 1997, EMBO J. 16: 578-587; and Yu, D. *et al.,* 1997, Genes Cells 2: 457-466).

For example, a recombinant Sendai virus vector carrying a foreign gene can be constructed as follows, according to descriptions in: Hasan, M. K. *et al.,* J. Gen. Virol. 78: 2813-2820, 1997; Kato, A. *et al.,* 1997, EMBO J. 16: 578-587; Yu, D. *et al.,* 1997, Genes Cells 2: 457-466; or the like.

First, a DNA sample comprising a cDNA sequence of an objective foreign gene is prepared. The DNA sample is preferably one that can be confirmed to be a single plasmid by electrophoresis at a concentration of 25 ng/µl or more. The following explains the case of using a *Not* I site to insert a foreign gene into a DNA encoding a viral genomic RNA, with reference to examples. When a *Not* I recognition site is included in a target cDNA nucleotide sequence, the base sequence is altered using site-directed mutagenesis or the like, such that the encoded amino acid sequence does not change, and the *Not* I site is preferably excised in advance. The objective gene fragment is amplified from this sample by PCR, and then recovered. By adding the *Not* I site to the 5' regions of a pair of primers, both ends of the amplified fragments become *Not* I sites. E-I-S sequences are designed to be included in primers such that, after a foreign gene is inserted into the viral genome, one E-I-S sequence each is placed between the ORF of the foreign gene, and either side of the ORFs of the viral genes.

For example, to guarantee cleavage with *Not* I*,* the forward side synthetic DNA sequence has a form in which any desired sequence of not less than two nucleotides (preferably four nucleotides not comprising a sequence derived from the *Not* I recognition site, such as GCG and GCC, and more preferably ACTT) is selected at the 5'-side, and a *Not* I recognition site gcggccgc is added to its 3'-side. To that 3'-side, nine arbitrary nucleotides, or nine plus a multiple of six nucleotides are further added as a spacer sequence. To the further 3' of this, a sequence corresponding to about 25 nucleotides of the ORF of a desired cDNA, including and counted from the initiation codon ATG, is added. The 3'-end of the forward side synthetic oligo DNA is preferably about 25 nucleotides, selected from the desired cDNA such that the final nucleotide becomes a G or C.

For the reverse side synthetic DNA sequence, no less than two arbitrary nucleotides (preferably four nucleotides not comprising a sequence derived from a *Not* I recognition site, such as GCG and GCC, and more preferably ACTT) are selected from the 5'-side, a *Not* I recognition site 'gcggccgc' is added to its 3'-side, and to that 3' is further added an oligo DNA insert fragment for adjusting the length. The length of this oligo DNA is designed such that the chain length of the *Not* I fragment of the final PCR-amplified product will become a multiple of six nucleotides (the so-called "rule of six"); Kolakofski, D., *et al.,* J. Virol. 72:891-899, 1998; Calain, P. and Roux, L., J. Virol. 67:4822-4830, 1993; Calain, P. and Roux, L., J. Virol. 67: 4822-4830, 1993). When adding an E-I-S sequence to this primer, to the 3'-side of the oligo DNA insertion fragment is added the complementary strand sequence of the Sendai virus S, I, and E sequences, preferably 5'-CTTTCACCCT-3' (SEQ ID NO: 1), 5'-AAG-3', and 5'-TTTTTCTTACTACGG-3' (SEQ ID NO: 2), respectively; and further to this 3'-side is added a complementary strand sequence corresponding to about 25 nucleotides, counted backwards from the termination codon of a desired cDNA sequence, whose length has been selected such that the final nucleotide of the chain becomes a G or C, to make the 3'-end of the reverse side synthetic DNA.

PCR can be performed by usual methods using Taq polymerase or other DNA polymerases. Objective amplified fragments are digested with *Not* I, and then inserted into the *Not* I site of plasmid vectors such as pBluescript. The nucleotide sequences of PCR products thus obtained are confirmed with a sequencer, and plasmids comprising the correct sequence are selected. The inserted fragment is excised from these plasmids using *Not* I*,* and cloned into the *Not* I site of a plasmid comprising genomic cDNA. A recombinant Sendai virus cDNA can also be obtained by inserting the fragment directly into the *Not* I site of a genomic cDNA, without using a plasmid vector.

For example, a recombinant Sendai virus genomic cDNA can be constructed according to methods described in the literature (Yu, D. *et al.,* Genes Cells 2: 457-466, 1997; Hasan, M. K. *et al.,* J. Gen. Virol. 78: 2813-2820, 1997). For example, an 18 bp spacer sequence (5'-(G)-CGGCCGCAGATCTTCACG-3') (SEQ ID NO: 3), comprising a *Not* I restriction site, is inserted between the leader sequence and the ORF of N protein of the cloned Sendai virus genomic cDNA (pSeV(+)), obtaining plasmid pSeV18⁺b(+), which comprises an auto-cleavage ribozyme site derived from the antigenomic strand of delta hepatitis virus (Hasan, M. K. *et al.,* 1997, J. General Virology 78: 2813-2820). A recombinant Sendai virus cDNA comprising a desired foreign gene can be obtained by inserting a foreign gene fragment into the *Not* I site of pSeV18⁺b(+).

A vector of this invention can be reconstituted by transcribing a DNA encoding a genomic RNA of a recombinant paramyxovirus thus prepared, in cells in the presence of the above-described viral proteins (L, P, and N). The present invention provides DNAs encoding the viral genomic RNAs of the vectors of this invention, for manufacturing the vectors of this invention. This invention also relates to the use of DNAs encoding the genomic RNAs of the vectors, in the manufacture of the vectors of this invention. The recombinant viruses can be reconstituted by methods known in the art (WO97/16539; WO97/16538; Durbin, A. P. *et al.,* 1997, Virology 235: 323-332; Whelan, S. P. *et al.,* 1995, Proc. Natl. Acad. Sci. USA 92: 8388-8392; Schnell. M. J. *et al.,* 1994, EMBO J. 13: 4195-4203; Radecke, F. *et al.,* 1995, EMBO J. 14: 5773-5784; Lawson, N. D. *et al.,* Proc. Natl. Acad. Sci. USA 92: 4477-4481; Garcin, D*. et al.,* 1995, EMBO J. 14: 6087-6094; Kato, A. *et al.,* 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). With these methods, minus strand RNA viruses including parainfluenza virus, vesicular stomatitis virus, rabies virus, measles virus, rinderpest virus, and Sendai virus can be reconstituted from DNA. The vectors of this invention can be reconstituted according to these methods. When a viral vector DNA is made F gene, HN gene, and/or M gene deficient, such DNAs do not form infectious virions as is. However, infectious virions can be formed by separately introducing host cells with these deficient genes, and/or genes encoding the envelope proteins of other viruses, and then expressing these genes therein.

Specifically, the viruses can be prepared by the steps of: (a) transcribing cDNAs encoding paramyxovirus genomic RNAs (negative strand RNAs), or complementary strands thereof (positive strands), in cells expressing N, P, and L proteins; and (b) harvesting culture supernatants thereof comprising the produced paramyxovirus. For transcription, a DNA encoding a genomic RNA is linked downstream of an appropriate promoter. The genomic RNA thus transcribed is replicated in the presence of N, L, and P proteins to form an RNP complex. Then, in the presence of M, HN, and F proteins, virions enclosed in an envelope are formed. For example, a DNA encoding a genomic RNA can be linked downstream of a T7 promoter, and transcribed to RNA by T7 RNA polymerase. Any desired promoter can be used as a promoter, in addition to those comprising a T7 polymerase recognition sequence. Alternatively, RNA transcribed *in vitro* may be transfected into cells.

Enzymes essential for the initial transcription of genomic RNA from DNA, such as T7 RNA polymerase, can be supplied by transducing the plasmid or viral vectors that express them, or, for example, by incorporating the RNA polymerase gene into a chromosome of the cell so as to enable induction of its expression, and then inducing expression at the time of viral reconstitution. Further, genomic RNA and viral proteins essential for vector reconstitution are supplied, for example, by transducing the plasmids that express them. In supplying these viral proteins, helper viruses such as the wild type or certain types of mutant paramyxovirus are used.

Methods for transducing DNAs expressing the genomic RNAs into cells include, for example, (i) methods for making DNA precipitates which target cells can internalize; (ii) methods for making complexes comprising DNAs that are suitable for internalization by target cells, and have a low-cytotoxic positive charge; and (iii) methods for using electric pulses to instantaneously create holes in the target cell membrane, which are of sufficient size for DNA molecules to pass through.

For (ii), various transfection reagents can be used. For example, DOTMA (Roche), Superfect (QIAGEN #301305), DOTAP, DOPE, DOSPER (Roche #1811169), and such can be cited. As (i), for example, transfection methods using calcium phosphate can be cited, and although DNAs transferred into cells by this method are internalized by phagosomes, a sufficient amount of DNA is known to enter the nucleus (Graham, F. L. and Van Der Eb, J., 1973, Virology 52: 456; Wigler, M. and Silverstein, S., 1977, Cell 11: 223). Chen and Okayama investigated the optimization of transfer techniques, reporting that (1) incubation conditions for cells and coprecipitates are 2 to 4% CO₂, 35°C, and 15 to 24 hours, (2) the activity of circular DNA is higher than linear DNA, and (3) optimal precipitation is obtained when the DNA concentration in the precipitate mixture is 20 to 30 µg/ml (Chen, C. and Okayama, H., 1987, Mol. Cell. Biol. 7: 2745). The methods of (ii) are suitable for transient transfections. Methods for performing transfection by preparing a DEAE-dextran (Sigma #D-9885 M.W. 5x 10⁵) mixture with a desired DNA concentration ratio have been known for a while. Since most complexes are decomposed in endosomes, chloroquine may also be added to enhance the effect (Calos, M. P., 1983, Proc. Natl. Acad. Sci. USA 80: 3015). The methods of (iii) are referred to as electroporation methods, and are used more in general than methods (i) or (ii) because they are not cell-selective. The efficiency of these methods is supposed to be good under optimal conditions for: the duration of pulse electric current, shape of the pulse, potency of electric field (gap between electrodes, voltage), conductivity of buffer, DNA concentration, and cell density.

Of the above three categories, the methods of (ii) are simple to operate and facilitates examination of many samples using a large amount of cells, making transfection reagents suitable for the transduction into cells of DNA for vector reconstitution. Preferably, the Superfect Transfection Reagent (QIAGEN, Cat No. 301305), or the DOSPER Liposomal Transfection Reagent (Roche, Cat No. 1811169) is used, but transfection reagents are not limited to these.

Specifically, virus reconstitution from cDNA can be carried out, for example, as follows:

In a plastic plate of about 6 to 24 wells, or a 100-mm Petri dish or the like, simian kidney-derived LLC-MK2 cells are cultured up to about 100% confluency, using minimum essential medium (MEM) comprising 10% fetal calf serum (FCS) and antibiotics (100 units/ml penicillin G and 100 µg/ml streptomycin). Then they are infected with, for example, two plaque forming units (PFU)/cell of the recombinant vaccinia virus vTF7-3, which expresses T7 RNA polymerase and has been inactivated by 20-minutes of UV irradiation in the presence of 1 µg/ml psoralen (Fuerst, T. R. *et al.,* Proc. Natl. Acad. Sci. USA 83: 8122-8126,1986; Kato, A. *et al.,* Genes Cells 1: 569-579, 1996). The amount of psoralen added and the UV irradiation time can be appropriately adjusted. One hour after infection, 2 to 60 µg, and more preferably 3 to 20 µg, of DNA encoding the genomic RNA of a recombinant Sendai virus is transfected along with the plasmids expressing trans-acting viral proteins essential for viral RNP production (0.5 to 24 µg of pGEM-N, 0.25 to 12 µg ofpGEM-P, and 0.5 to 24 µg of pGEM-L) (Kato, A. *et al.,* Genes Cells 1: 569-579, 1996), using the lipofection method or such with Superfect (QIAGEN). For example, the ratio of the amounts of expression vectors encoding the N, P, and L proteins is preferably 2:1:2, and the plasmid amounts are appropriately adjusted in the range of 1 to 4 µg of pGEM-N, 0.5 to 2 µg of pGEM-P, and 1 to 4 µg of pGEM-L.

The transfected cells are cultured, as desired, in serum-free MEM comprising 100 µg/ml of rifampicin (Sigma) and cytosine arabinoside (AraC), more preferably only 40 µg/ml of cytosine arabinoside (AraC) (Sigma). Optimal drug concentrations are set so as to minimize cytotoxicity due to the vaccinia virus, and to maximize virus recovery rate (Kato, A. *et al.,* 1996, Genes Cells 1: 569-579). After culturing for about 48 to 72 hours after transfection, cells are harvested, and then disintegrated by repeating freeze-thawing three times. LLC-MK2 cells are re-infected with the disintegrated materials comprising RNP, and cultured. Alternatively, the culture supernatant is recovered, added to a culture solution of LLC-MK2 cells to infect them, and the cells are then cultured. Transfection can be conducted by, for example, forming a complex with lipofectamine, polycationic liposome, or the like, and transducing the complex into cells. Specifically, various transfection reagents can be used. For example, DOTMA (Roche), Superfect (QIAGEN #301305), DOTAP, DOPE, and DOSPER (Roche #1811169) may be cited. In order to prevent decomposition in the endosome, chloroquine may also be added (Calos, M. P., 1983, Proc. Natl. Acad. Sci. USA 80: 3015). In cells transduced with RNP, viral gene expression from RNP and RNP replication progress, and the vector is amplified. By diluting the viral solution thus obtained (for example, 10⁶-fold), and then repeating the amplification, the vaccinia virus vTF7-3 can be completely eliminated. Amplification is repeated, for example, three or more times. Vectors thus obtained can be stored at -80°C. In order to reconstitute a viral vector having no replication ability and lacking a gene encoding an envelope protein, LLC-MK2 cells expressing the envelope protein may be used for transfection, or a plasmid expressing the envelope protein may be cotransfected. Alternatively, a defective type viral vector can be amplified by culturing the transfected cells overlaid with LLK-MK2 cells expressing the envelope protein (see WO00/70055 and WO00/70070).

Titers of viruses thus recovered can be determined, for example, by measuring CIU (Cell-Infected Unit) or hemagglutination activity (HA) (WO00/70070; Kato, A. *et al.,* 1996, Genes Cells 1: 569-579; Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). Titers of vectors carrying GFP (green fluorescent protein) marker genes and the like can be quantified by directly counting infected cells, using the marker as an indicator (for example, as GFP-CIU). Titers thus measured can be treated in the same way as CIU (WO00/70070).

As long as a viral vector can be reconstituted, the host cells used in the reconstitution are not particularly limited. For example, in the reconstitution of Sendai virus vectors and such, cultured cells such as LLC-MK2 cells and CV-1 cells derived from monkey kidney, BHK cells derived from hamster kidney, and cells derived from humans can be used. By expressing suitable envelope proteins in these cells, infectious virions comprising the envelope can also be obtained. Further, to obtain a large quantity of a Sendai virus vector, a viral vector obtained from an above-described host can be infected to embrionated hen eggs, to amplify the vector. Methods for manufacturing viral vectors using hen eggs have already been developed (Nakanishi, *et al.,* ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for nine to twelve days at 37 to 38°C to grow an embryo. After the viral vector is inoculated into the allantoic cavity, the egg is cultured for several days (for example, three days) to proliferate the viral vector. Conditions such as the period of culture may vary depending upon the recombinant Sendai virus being used. Then, allantoic fluids comprising the vector are recovered. Separation and purification of a Sendai virus vector from allantoic fluids can be performed according to a usual method (Tashiro, M., "Virus Experiment Protocol," Nagai, Ishihama, ed., Medical View Co., Ltd., pp. 68-73, (1995)).

For example, the construction and preparation of Sendai virus vectors defective in F gene can be performed as described below (see WO00/70055 and WO00/70070).

### <1> Construction of a genomic cDNA of an F-gene defective Sendai virus, and a plasmid expressing F gene

A full-length genomic cDNA of Sendai virus (SeV), the cDNA of pSeV 18⁺ b (+) (Hasan, M. K. *et al.,* 1997, J. General Virology 78: 2813-2820) ("pSeV18⁺ b (+)" is also referred to as "pSeV18⁺"), is digested with SphI/KpnI to recover a fragment (14673 bp), which is cloned into pUC 18 to prepare plasmid pUC18/KS. Construction of an F gene-defective site is performed on this pUC18/KS. An F gene deficiency is created by a combination of PCR-ligation methods, and, as a result, the F gene ORF (ATG-TGA = 1698 bp) is removed. Then, for example, atgcatgccggcagatga (SEQ ID NO: 4) is ligated to construct an F gene-defective type SeV genomic cDNA (pSeV18⁺/ΔF). A PCR product formed in PCR by using the pair of primers [forward: 5'-gttgagtactgcaagagc/SEQ ID NO: 5, reverse: 5'-tttgccggcatgcatgtttcccaaggggagagttttgcaacc/SEQ ID NO: 6] is connected upstream of F, and a PCR product formed using the pair of primers [forward: 5'-atgcatgccggcagatga/SEQ ID NO: 7, reverse: 5'-tgggtgaatgagagaatcagc/SEQ ID NO: 8] is connected downstream of F gene at EcoT22I. The plasmid thus obtained is digested with SacI and SalI to recover a 4931 bp fragment of the region comprising the F gene-defective site, which is cloned into pUC18 to form pUC18/dFSS. This pUC18/dFSS is digested with DraIII, the fragment is recovered, replaced with the DraIII fragment of the region comprising the F gene of pSeV18⁺, and ligated to obtain the plasmid pSeV18⁺/ΔF.

A foreign gene is inserted, for example, into the *Nsi* I and *Ngo* MIV restriction enzyme sites in the F gene-defective site of pUC18/dFSS. For this, a foreign gene fragment may be, for example, amplified using an *Nsi* I-tailed primer and an *Ngo* MIV-tailed primer.

### <2> Preparation of helper cells that induce SeV-F protein expression

To construct an expression plasmid of the Cre/loxP induction type that expresses the Sendai virus F gene (SeV-F), the SeV-F gene is amplified by PCR, and inserted to the unique *Swa* I site of the plasmid pCALNdlw (Arai, T. *et al.,* J. Virology 72, 1998, p1115-1121), which is designed to enable the inducible expression of a gene product by Cre DNA recombinase, thus constructing the plasmid pCALNdLw/F.

To recover infectious virions from the F gene-defective genome, a helper cell line expressing SeV-F protein is established. The monkey kidney-derived LLC-MK2 cell line, which is commonly used for SeV proliferation, can be used as the cells, for example. LLC-MK2 cells are cultured in MEM supplemented with 10% heat-treated inactivated fetal bovine serum (FBS), penicillin G sodium (50 units/ml), and streptomycin (50 µg/ml) at 37°C in 5% CO₂. Since the SeV-F gene product is cytotoxic, the above-described plasmid pCALNdLw/F, which was designed to enable inducible expression of the F gene product with Cre DNA recombinase, is transfected to LLC-MK2 cells for gene transduction by the calcium phosphate method (using a mammalian transfection kit (Stratagene)), according to protocols well known in the art.

The plasmid pCALNdLw/F (10 µg) is transduced into LLC-MK2 cells grown to 40% confluency using a 10-cm plate, and the cells are then cultured in MEM (10 ml) comprising 10% FBS, in a 5% CO₂ incubator at 37°C for 24 hours. After 24 hours, the cells are detached and suspended in the medium (10 ml). The suspension is then seeded into five 10-cm dishes, 5 ml into one dish, 2 ml each into two dishes, and 0.2 ml each into two dishes, and cultured in MEM (10 ml) comprising G418 (GIBCO-BRL) (1200 µg/ml) and 10% FBS. The cells were cultured for 14 days, exchanging the medium every two days, to select cell lines stably transduced with the gene. The cells grown from the above medium that show G418 resistance are recovered using a cloning ring. Culture of each clone thus recovered is continued in 10-cm plates until confluent.

After the cells have grown to confluency in a 6-cm dish, F protein expression is induced by infecting the cells with Adenovirus AxCANCre, for example, at MOI = 3, according to the method of Saito, *et al.* (Saito *et al.,* Nucl. Acids Res. 23: 3816-3821 (1995); Arai, T. *et al.,* J. Virol 72, 1115-1121 (1998)).

### <3> Reconstitution and amplification of F-gene defective Sendai virus (SeV)

The above-described plasmid pSeV 18+/ΔF, into which a foreign gene has been inserted, is transfected to LLC-MK2 cells as follows: LLC-MK2 cells are seeded at 5x 10⁶ cells/dish into 100-mm dishes. When genomic RNA transcription is carried out with T7·RNA polymerase, cells are cultured for 24 hours, then infected at an MOI of about two for one hour at room temperature, with recombinant vaccinia virus expressing T7.RNA polymerase, which has been treated with psoralen and long-wave ultraviolet rays (365 nm) for 20 minutes (PLWUV-VacT7: Fuerst, T. R. *et al.,* Proc. Natl. Acad. Sci. USA 83, 8122-8126 (1986)). For the ultraviolet ray irradiation of vaccinia virus, for example, an UV Stratalinker 2400 equipped with five 15-watt bulbs can be used (catalogue No. 400676 (100V), Stratagene, La Jolla, CA, USA). The cells are washed with serum-free MEM, then an appropriate lipofection reagent is used to transfect the cells with a plasmid expressing the genomic RNA, and expression plasmids expressing the N, P, L, F, and HN proteins of *Paramyxovirus* respectively. The ratio of the amounts of these plasmids can be preferably set as 6:2:1:2:2:2, in this order, though not limited thereto. For example, a genomic RNA-expressing plasmid as well as expression plasmids expressing the N, P, L, and F plus HN proteins (pGEM/NP, pGEM/P, pGEM/L, and pGEM/F-HN; WO00/70070, Kato, A. *et al.,* Genes Cells 1, 569-579 (1996)) are transfected at an amount ratio of 12 µg:12 µg: 4 µg: 2 µg: 4 µg:4 µg per dish, respectively. After culturing for several hours, the cells are washed twice with serum-free MEM, and cultured in MEM comprising 40 µg/ml of cytosine β-D-arabinofuranoside (AraC: Sigma, St. Louis, MO) and 7.5 µg/ml of trypsin (Gibco-BRL, Rockville, MD). These cells are recovered, and the pellets are suspended in Opti-MEM (10⁷ cells/ml). Suspensions are freeze-thawed three times, mixed with lipofection reagent DOSPER (Boehringer Mannheim) (10⁶ cells/25 µl DOSPER), stood at room temperature for 15 minutes, transfected to the above-described cloned F-expressing helper cells (10⁶ cells/well in a 12-well-plate), and cultured in serum-free MEM (comprising 40 µg/ml AraC and 7.5 µg/ml trypsin) to recover the supernatant. Viruses lacking a gene other than F, for example, the HN or M gene, can also be prepared by methods similar to this.

For example, when preparing a gene-deficient viral vector, two or more types of vectors containing viral genomes lacking different viral genes can be introduced into the same cell, and the viral proteins absent from one vector can be supplied through the expression of the other vectors. These vectors complement each other and form infectious virions, thereby turning on the replication cycle for amplification of viral vectors. That is, two or more types of vectors of this invention can be injected into a cell in combinations of complementary viral proteins, to produce, on a large scale and at a low cost, mixtures of viral vectors lacking the respective viral genes . Compared to viruses that do not lack viral genes, these viruses have a reduced genome size because they are deficient in viral genes, and are thus able to carry large-sized foreign genes. These viral gene-deficient viruses do not have proliferation ability and are diluted extracellularly. This makes maintenance of coinfection difficult, resulting in sterility, which is advantageous from the viewpoint of managing enviromental release of these viruses.
Foreign genes to be transduced by the paramyxoviruses of this invention are not particularly limited, and examples of naturally occuring proteins are hormones, cytokines, growth factors, receptors, intracellular signal transduction molecules, enzymes, peptides, etc. Proteins may be secretory proteins, membrane proteins, cytoplasmic proteins, nuclear proteins, etc. Examples of artificial proteins are fusion proteins such as chimeric toxins, dominant negative proteins (including soluble receptor molecules and membrane-bound type receptors), deletant cell adhesion molecules, cell surface molecules, and such. Artificial proteins may be proteins with added secretory signals, membrane localization signals, nuclear localization signals, etc. By expressing a transgene such as an anti-sense RNA molecule, RNA-cleaving ribozyme or such, the function of a specific gene expressed in T cells can be suppressed. Gene therapy can be performed by administering, as a foreign gene, a viral vector prepared using a therapeutic gene for a disease . The viral vectors of this invention can be applied in gene therapy through direct or indirect (*ex vivo*) administration of the vectors by gene expression methods that express, for example, foreign genes with promising therapeutic effects or endogenous genes whose supply is insufficient within the patient's body. Further, the methods of this invention can also be applied to gene therapy vectors in regenerative medicine.

When, after administering a replicative paramyxovirus vector to an individual or cell, the proliferation of the viral vector must be restrained due to completion of treatment and such, it is also possible to specifically restrain only the proliferation of the viral vector, with no damage to the host, by administering an RNA-dependent RNA polymerase inhibitor.

According to the methods for producing a virus described herein, the viral vectors of this invention can be released into the culture medium of virus-producing cells, for example, at a titer of 1x 10⁵ CIU/ml or more, preferably 1x 10⁶ CIU/ml or more, more preferably 5x 10⁶ CIU/ml or more, more preferably 1x 10⁷ CIU/ml or more, more preferably 5x 10⁷ CIU/ml or more, more preferably 1x 10⁸ CIU/ml or more, and more preferably 5x 10⁸ CIU/ml or more. Viral titers can be measured by a method described in this description or other methods (Kiyotani, K. *et al.,* Virology 177(1), 65-74 (1990); WO00/70070).

The recovered paramyxovirus vectors can be purified to become substantially pure. Purification can be performed by purification and separation methods known in the art, including filtration, centrifugal separation, and column purification, or any combinations thereof. "Substantially pure" means that a viral vector accounts for a major proportion of a sample in which the viral vector exists as a component. Typically, a substantially pure viral vector can be identified by confirming that the proportion of proteins derived from the viral vector is 10% or more of all of the proteins in a sample, preferably 20% or more, more preferably 50% or more, preferably 70% or more, more preferably 80% or more, and further more preferably 90% or more (excluding, however, proteins added as carriers or stabilizers). Examples of specific methods for purifying paramyxoviruses are those that use cellulose sulfate ester or cross-linked polysaccharide sulfate ester (Examined Published Japanese Patent Application No. (JP-B) Sho 62-30752, JP-B Sho 62-33879, and JP-B Sho 62-30753), and those that involve adsorption onto polysaccharides comprising fucose sulfate and/or degradation products thereof (WO97/32010).

In preparing compositions comprising a vector, the vector can be combined with a pharmaceutically acceptable carrier or vehicle of choice, as necessary. "A pharmaceutically acceptable carrier or vehicle" means a material that can be administered together with the vector which does not significantly inhibit gene transduction via the vector. For example, a vector can be appropriately diluted with physiological saline, phosphate-buffered saline (PBS), or culture medium to form a composition. When a vector is grown in hen eggs or the like, the "pharmaceutically acceptable carrier or vehicle" may comprise allantoic fluids. Further, compositions comprising a vector may include carriers or vehicles such as deionized water and 5% dextrose aqueous solution. Furthermore, compositions may comprise, besides the above, plant oils, suspending agents, surfactants, stabilizers, biocides, and so on. The compositions can also comprise preservatives or other additives. The compositions comprising the vectors of this invention are useful as reagents and medicines.

Gene transduction into T cells using the vectors of this invention is expected to be applied to gene therapy for various disorders. Such gene therapy can be performed to, for example, correct abberant cellular expression of a gene due to the deficiency thereof, impart a novel function to a cell via introduction of a foreign gene, or suppress an undesirable function in a cell by introducing a gene with suppressive action towards a particular gene.

The methods of this invention are useful in, for example, suppressing rejection responses in autoimmune diseases and others. For example, rejection responses can be expected to be controlled by suppressing alloreactions of T cells *in vivo* or inducing suppressive dendritic cells. This involves using an established activated T cell line that recognizes an alloantigen or a major antigen that is causing the autoimmune disease, and actively expressing suppressive cytokines such as IL-10 in the established T cell line according to the methods of this invention. Further, cancer therapy that utilizes T cell gene transduction using the methods of this invention is also expected. For example, when a vector carrying a vascular proliferation-suppressing gene is transduced into T cells that recognize a tumor-specific antigen, suppressive effects of local tumor growth are expected. Alternatively, in the case of demyelinating diseases such as Multiple Sclerosis, disease progression can be expected to be controlled by using T cells activated with a target antigen to transduce a gene, such as the PDFG gene (platelet derived growth factor-A) which is capable of differentiating oligodendrocytes from stem cells, thereby inducing the local regeneration of oligodendrocytes (Vincent, K.T. *et al.,* Journal of Neuroimmunology, 2000, 107: 226-232). In addition, it is possible to apply the methods of this invention to all diseases and injuries, where the therapeutic effects through gene transduction using antigen-activated T cells or antigen-nonspecific activated T cells (T cells activated using an antibody or mitogen) are expected.

Host-derived factors such as interferons (IFNs) affect the transcription of paramyxovirus-transduced genes (Kato, A. *et al.,* J. Virol., 2001, 75: 3802-3810). Since large amounts of IFNs are produced from T cell lines stimulated with alloantigens, IFNs such as IFN-γ are likely to affect the transcription of genes harbored on paramyxovirus vectors (Biron, C.A. and Sen, G.C., Interferons and other cytokine. *In*: Fields BN, Knipe DM, Howley PM, (eds). Fields of virology. Vol. 2. Lippincott - Raven Publishers: Philadelphia, 1996. 321-351). In fact, the present inventors discovered that the alloactivated T cell lines from IFN-γ receptor-deficient mice maintain relatively high levels of EGFP expression for more than 3 weeks. Therefore, in T cell-directed gene therapy that requires high-level expression of a target gene, suppression of IFN-γ signal transduction is likely to be effective.

Vector dose may vary depending upon the disorder, body weight, age, gender, and symptoms of the patient, as well as the purpose of administration, form of the composition to be administered, administration method, gene to be transduced, and so on; however, those skilled in the art can appropriately determine dosages. Administration route can be appropriately selected. Administration can also be performed locally or systemically. Doses of the vector are preferably administered in a pharmaceutically acceptable carrier in a range of preferably about 10⁵ CIU/ml to about 10¹¹ CIU/ml, more preferably about 10⁷ CIU/ml to about 10⁹ CIU/ml, most preferably about 1x 10⁸ CIU/ml to about 5x 10⁸ CIU/ml. In humans, a single dose is preferably in the range of 2x 10⁵ CIU to 2x 10¹¹ CIU, and can be administered once or more, so long as the side effects are within a clinically acceptable range. The same applies to the number of administrations per day. With animals other than humans, for example, the above-described doses can be converted based on the body weight ratio or volume ratio of a target site for administration (e.g. average values) between the objective animal and humans, and the converted doses can be administered to the animals. In the case of an *ex vivo* administration, the vector is brought into contact with T cells *in vitro* (for example, in a test tube or Petri dish). Vectors are administered to T cells at an MOI in the range of preferably 1 to 500, more preferably 2 to 300, and further more preferably 3 to 200. Subjects to whom compositions comprising the vectors of this invention are administered include all mammals, such as humans, monkeys, mice, rats, rabbits, sheep, cattle, and dogs.

### Brief Description of the Drawings

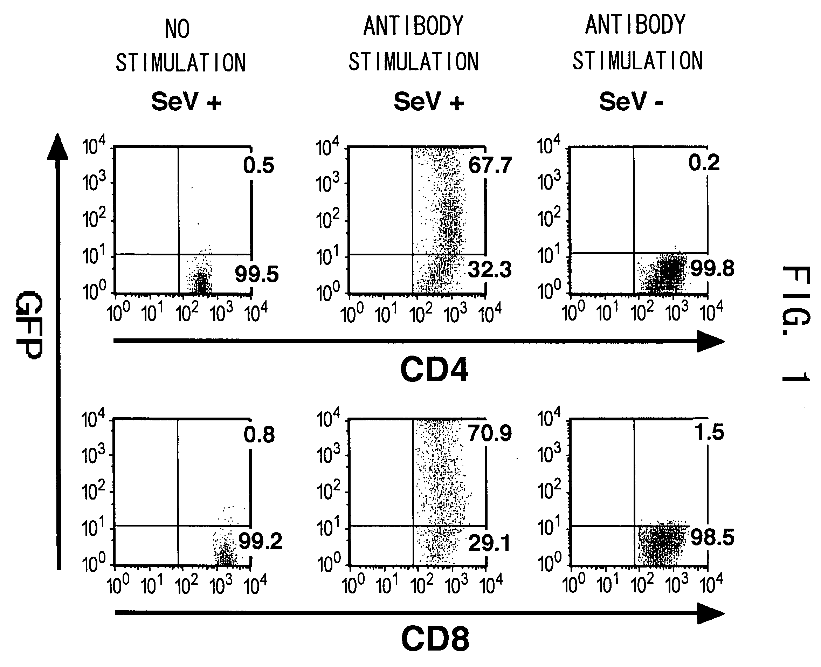

Fig. 1 is a series of dot plot graphs representing the gene transduction efficency of SeV into murine T cells (activated or naive T cells). Murine lymphocytes were cultured in the presence or absence of SeV-EGFP (2.5x 10⁷ PFU) (MOI=62.5) for two days, and cells were recovered and stained with APC-conjugated anti-CD3 and PE-conjugated anti-CD4 (upper panel) or CD8 antibodies (lower panel). Dot plots represent the expression of CD4 or CD8 and GFP among viable CD3⁺ CD4⁺- or CD3⁺ CD8⁺-lymphocytes. In the right upper corners of the respective quadrants, gene transduction efficiency was shown as the percentage of EGFP-positive cells. Left panel: cells were cultured in non-Ab-coated wells. Central panel: cells were cultured in wells coated with activating antibodies (anti-CD3- and anti-CD28-antibodies). Right panel: cells were cultured in wells coated with activating antibodies (anti-CD3- and anti-CD28-antibodies) in the absence of SeV-EGFP. Similar data were obtained from cells cultured with luciferase-expressing SeV-luci (negative control). Reproducible data were obtained from more than four independent experiments.

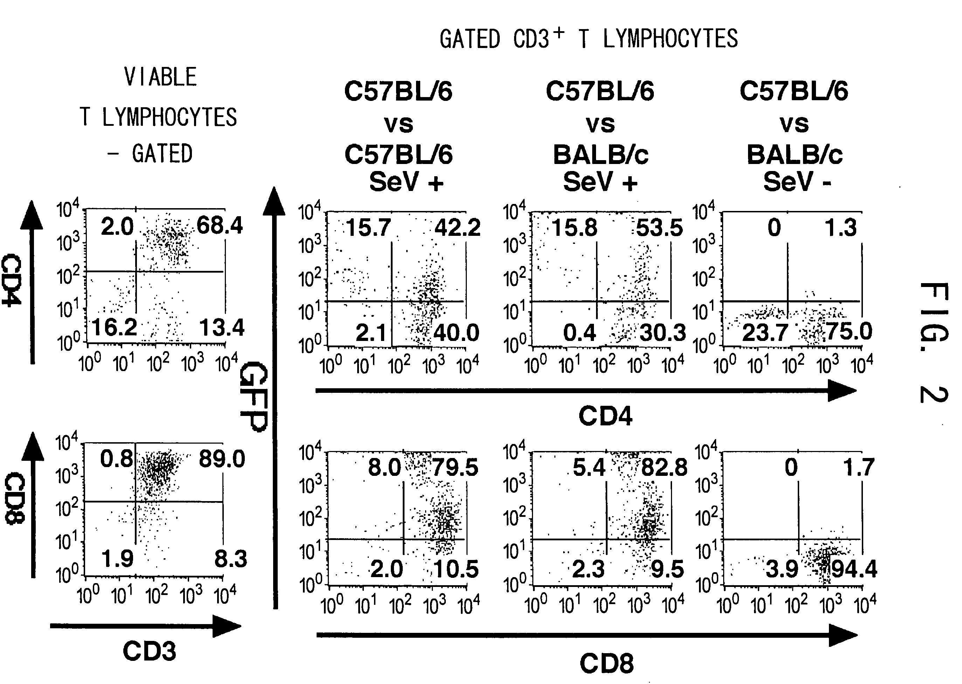

Fig. 2 is a series of dot plots representing the efficiency of SeV-mediated gene transduction into T cell lines. T cell lines were cultured in the presence or absence of SeV-EGFP (2.5x 10⁷ PFU) (MOI=62.5) for two days, and the recovered cells were stained with APC-conjugated anti-CD3 and PE-conjugated anti-CD4 (upper panel) or anti-CD8 antibodies (lower panel). Dot plots in the leftmost panel represent the expression of CD3 and CD4 or CD8 among gated (sorted) viable lymphocytes, showing the percentages of lymphocytes in the respective four quadrants. Dot plots in the other panels represent EGFP expression in CD4-T or CD8-T cells among gated CD3-positive viable lymphocytes. In the second panel from the left, cells were cultured with irradiated B6 stimulators (stimulator cells). In the third panel, cells were cultured with irradiated Balb/c stimulators (stimulator cells). In the last panel, cells were cultured with irradiated Balb/c stimulators (stimulator cells) without SeV-EGFP. Reproducible data were obtained from more than four independent experiments.

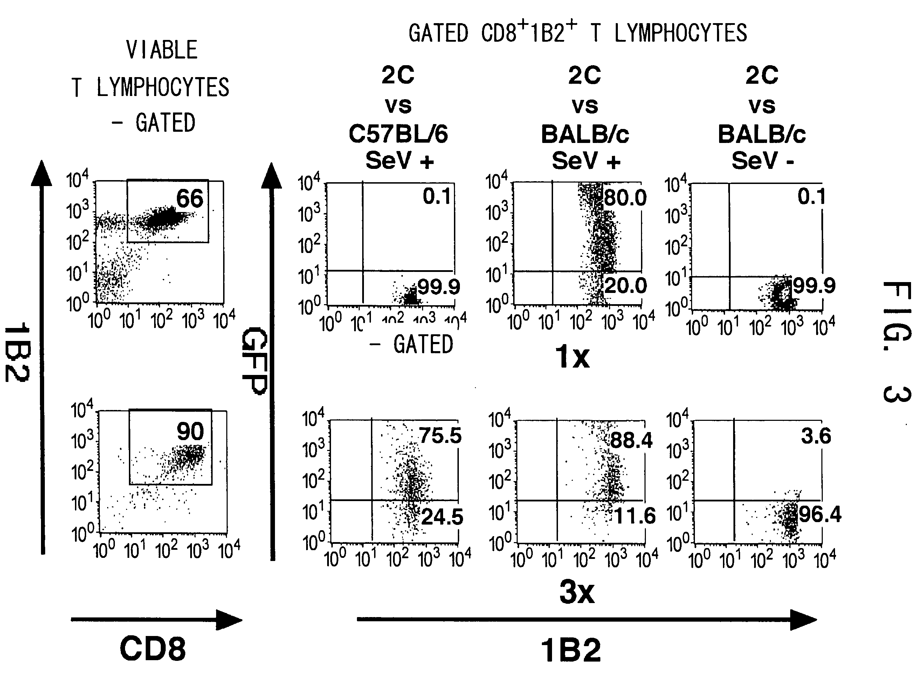

Fig. 3 is a series of dot plots representing the gene transduction efficiency under alloantigen-specific activation of naive T cells and T cell lines. Naive 2C lymphocytes (upper panel: with the letter "1x") or 2C T cell lines (lower panel: with the letter "3x") were cultured in the presence or absence of SeV-EGFP (2.5x 10⁷ PFU) for two days. Cells were recovered and stained with APC-conjugated anti-CD8 and biotinylated anti-clonotypic T cell receptor mAb (1B2), followed by PE streptavidin. The leftmost panel represents the percentage of CD8⁺ 1B2⁺ T cells among gated viable lymphocytes. Dot plots in the other panels respresent the EGFP expression of viable clonotypic T cells. In the second panel from the left, cells were cultured with irradiated B6 stimulators (stimulator cells). In the third panel, cells were cultured with irradiated Balb/c stimulators (stimulator cells). In the last panel, cells were cultured with irradiated Balb/c stimulators (stimulator cells) without SeV-EGFP.
   Reproducible data were obtained from two independent experiments.

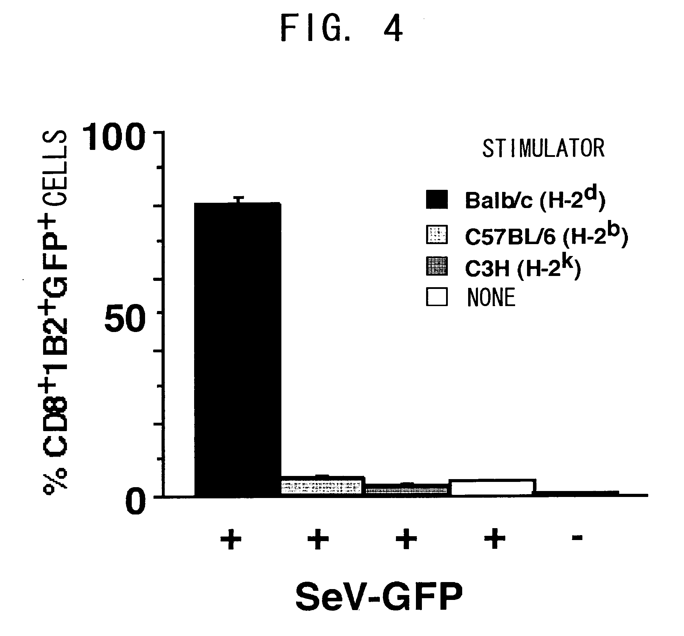

Fig. 4 is a bar graph representing the effect of bystander activation on SeV-mediated gene transduction. In the presence (indicated by plus sign below the X axis) or absence (indicated by minus sign below the X axis) of SeV-EGFP (2.5x 10⁷ PFU), 50 µl of 2C naive lymphocytes (1x 10⁷/ml) and 50 µl of B6 naive lymphocytes (1x 10⁷/ml) were stimulated with 100 µl each of irradiated Balb/c (solid bar), B6 (light shaded bar), or C3H (dark shaded bar) lymphocytes (1x 10⁷/ml), or without lymphocytes (open bar) for two days. The percentage of EGFP-positive cells among gated viable CD8⁺ 1B2⁺ T cells was obtained. Y axis represents the percentage of EGFP-positive clonotypic cells. Each data was shown as the mean percentage ± SEM (Standard Error of the Means) of triplicate wells (n=3), and similar results were observed in two independent experiments. There was a statistically significant difference (p<0.01) between 2C T cells stimulated with C3H and those stimulated with Balb/c, but not between 2C T cells stimulated with C3H and those stimulated with B6. Statistical significance was determined using the one-way anaylysis of variance and Fisher's PLSD test.

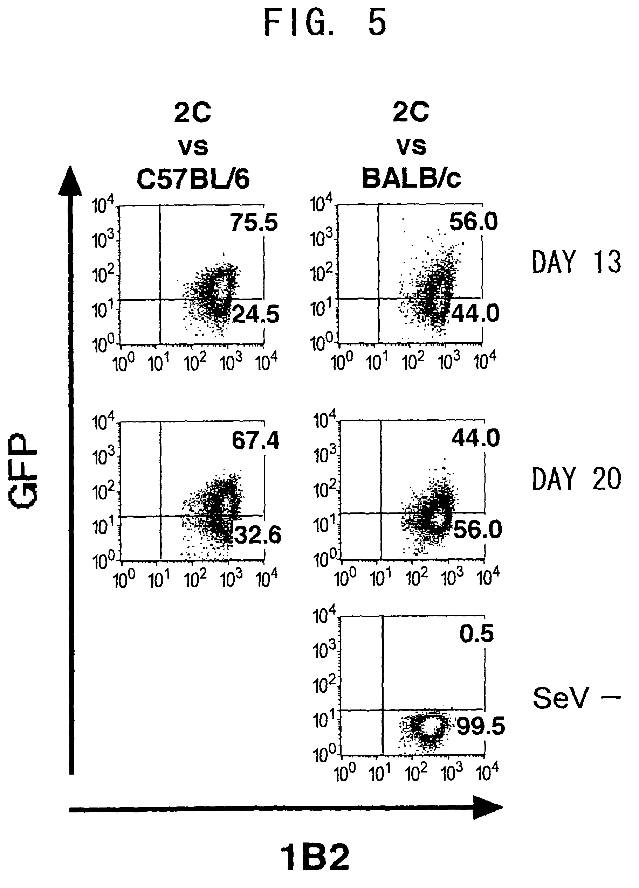

Fig. 5 is a series of dot plots representing the maintenance of GFP expression in T cells transduced with SeV-EGFP. In the presence of SeV-EGFP, T cell lines from 2C-tg mice were stimulated with irradiated B6 (left column) or Balb/c (right column) lymphocytes for six days. The transduced T cells were washed with fresh media, and then re-stimulated with irradiated B6 or Balb/c stimulator in the absence of SeV for six or seven days. Dot plots show the EGFP expression of viable clonotypic T cells. The number in the upper-right corner of the respective quadrants represents the percentage of EGFP-positive or negative clonotypic T cells at day 13 (top panel) or day 20 (middle panel). Data from the uninfected 2C T cell line (negative control) stimulated for 20 days were shown (bottom panel). These data are representative of two separate experiments.

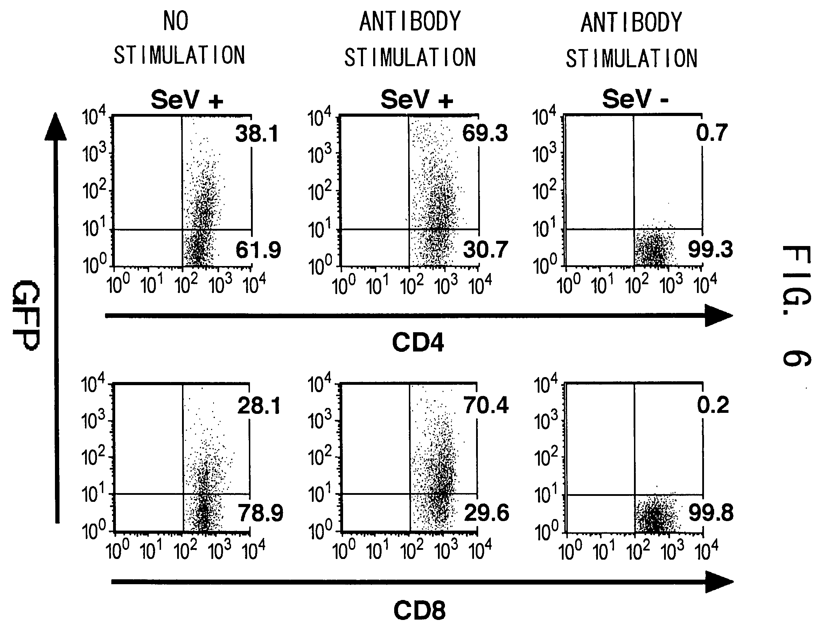

Fig. 6 is a series of dot plots representing the efficiency of SeV-mediated gene transduction into human T cells (activated or naive T cells). 200 µl of human lymphocytes (4x 10⁶/ml) were cultured in the presence or absence of SeV-EGFP (2.5x 10⁷ PFU) (MOI=31) for two days. Cells were recovered and stained with APC-conjugated anti-CD3 and PE-conjugated anti-CD4 (top panel) or CD8 antibodies (bottom panel). Dot plots represent the expression of CD4 or CD8 and GFP among viable CD3⁺ CD4⁺ or CD3⁺ CD8⁺ T lymphocytes, respectively. Gene transduction efficiency was expressed as the relative ratio of EGFP-positive cells among T cells shown in the plot. Left panel: cells were cultured in non-Ab-coated wells. Middle panel: cells were cultured in wells coated with activating antibodies (anti-CD3 and anti-CD28 antibodies). Right panel: cells were cultured in wells coated with activating antibodies (anti-CD3- and anti-CD28-antibodies) without SeV-EGFP. Similar data were obtained from cells cultured with SeV-luci (negative control). The numbers in the righthand corners of the respective quadrants represent the percentages of the individual populations. Reproducible data were obtained from more than four independent experiments.

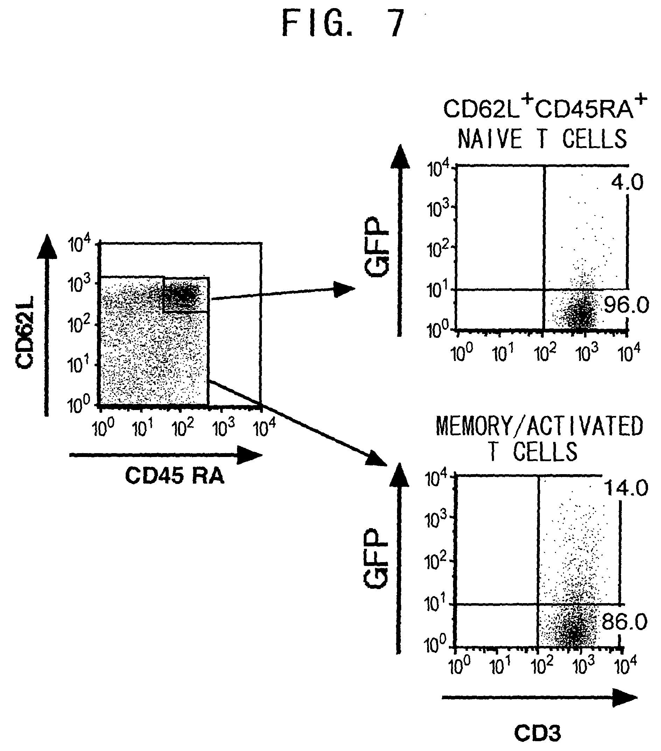

Fig. 7 is a series of dot plots representing the gene transduction efficiency into human naive or memory/activated T cells. The newly isolated T cells were cultured with SeV-EGFP in non-Ab-coated wells for two days. Cells were recovered and stained with APC-conjugated anti-CD62L, PE-conjugated anti-CD3, and biotinylated anti-CD45RA antibodies, and then with streptavidin-PerCP. In the left panel, dot plots represent the expression of CD62L and CD45RA in gated (sorted) viable CD3-positive T cells. The right panel dot plots represent the expression of CD3 and EGFP in CD62L^{high} and CD45RA^{high} T cells, which are either naive T cells (top panel) or memory/activated T cells (bottom panel). Reproducible data were obtained from three experiments using samples from healthy donors.

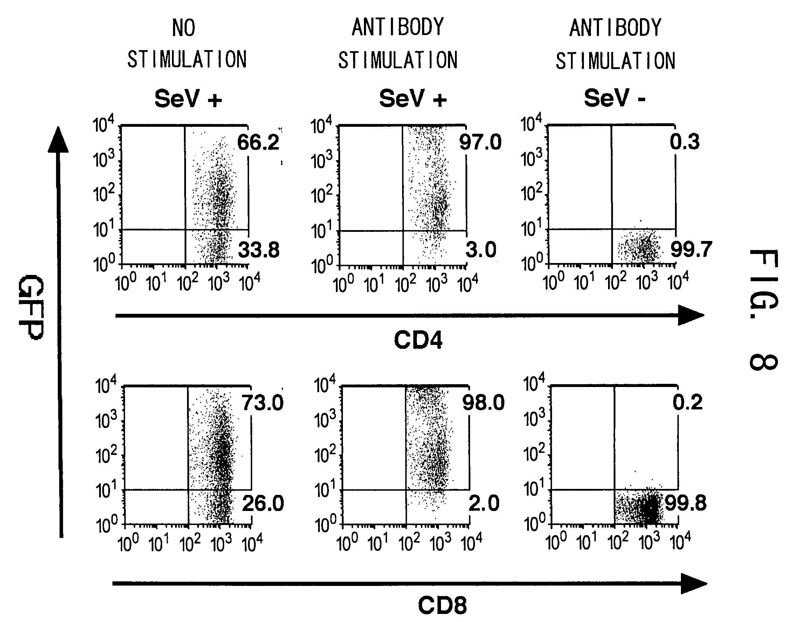

Fig. 8 is a series of dot plots representing the gene transduction efficiency into human T cell lines. The human T cell lines were cultured, as described in Fig. 6, in the presence or absence of SeV-EGFP for two days, and analyzed as described in Fig. 6. Percentages of EGFP expression in CD4 (top panel) and CD8 (bottom panel) T cells were shown. Left panel: cells were cultured in non-Ab-coated wells. Central panel: cells were cultured in wells coated with antibodies (human anti-CD3 and anti-CD28 antibodies). Right panel: cells were cultured in wells coated with antibodies (human anti-CD3- and anti-CD28-antibodies) in the absence of SeV-EGFP.

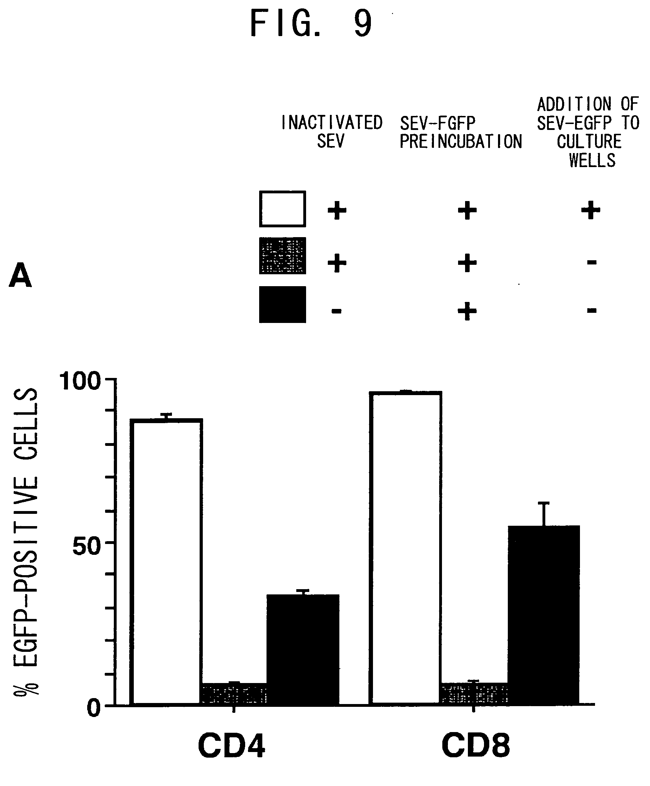

Fig. 9 represents bar graphs showing the assessment of SeV entry into naive or activated T cells. After B6 lymphocytes (4x 10⁶/ml) were incubated in the presence (open or shaded bar) or absence (solid bar) of UV-inactivated SeV-luci for 30 min at 37°C, the cells were thoroughly washed and incubated with SeV-EGFP (MOI=62.5) for 30 min at 37°C. After the cells were washed three times, 200 µl of the cell suspension (2x 10⁶/ml) was cultured in activated wells coated with mouse anti-CD3 and anti-CD28 antibodies in the absence of the virus for two days. As a positive control, the prepared cells were cultured with SeV-EGFP (2.5x 10⁷ PFU) in activated wells for two days (open bar). Cells were recovered and stained with APC-conjugated anti-CD3 and PE-conjugated anti-CD8 antibodies. The percentage of EGFP-positive cells among gated (sorted) viable CD3⁺ CD4⁺ or CD3⁺ CD8⁺ T cells was obtained. The Y-axis shows the percentage of EGFP-positive CD4 (three bars on the left) or CD8 (three bars on the right) T cells. Respective data were expressed as the mean percentage ± SEM of triplicate wells (n=3). There were statistically significant differences among the individual populations (p<0.01). Statistical significance was determined by the one-way analysis of variance and Fisher's PLSD test.

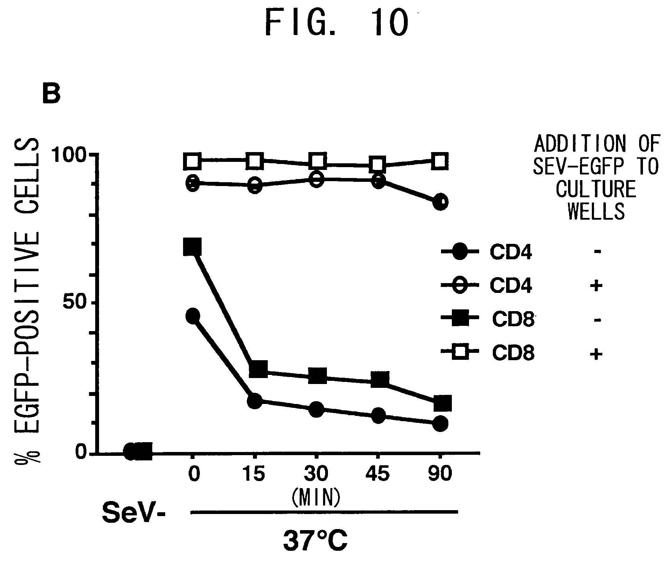

Fig. 10 represents a graph showing the assessment of SeV entry into naive or activated T cells. After 30 minutes of incubation at 4°C in the absence (the leftmost scale along the X axis) or presence (other scales on the X axis) of SeV-EGFP (MOI=100) for 30 min at, B6 lymphocytes were washed thoroughly and incubated without SeV for 0, 15, 30, 45, or 90 min at 37°C. After three washes, the cells incubated for the indicated periods were cultured in activated wells coated with mouse anti-CD3 and anti-CD28-antibodies for two days. The cells were recovered and stained as described in Fig. 9 (CD4 T cells were represented by solid circles and CD8 T cells by solid squares). As a positive control, the prepared cells were cultured with SeV-EGFP (2.5x 10⁷PFU) (MOI=62.5) (CD4 T cells were represented by open circles and CD8 T cells by open squares). Values were expressed as the mean percentage ± SEM of triplicate wells (n=3). In both CD4 and CD8 T cells, there was a statistically significant difference (p<0.01) between incubation at 37°C for 0 min and incubation for 15 min. Further, there was also a statistically significant difference (p<0.01) between incubation at 37°C for 0 min and incubation for 90 min. Statistical significance was determined by the one-way analysis of variance and Fisher's PLSD test.

### Best Mode for Carrying Out the Invention

Hereinafter, the present invention will be explained in more detail with reference to examples, but it is not to be construed as being limited thereto. All references cited in this description are incorporated into the description as parts thereof. Statistical significance was determined by the one-way analysis of variance and Fisher's PLSD test with a statistical significance of P<0.05.

### Animals

Inbred female C57BL/6 (H-2^{b}) (abbreviated B6) mice, C3H (H-2^{k}) mice, and Balb/c (H-2^{d}) mice of Charles River grade mice were purchased from KBT Oriental (Tosu, Japan). 2C transgenic mice (2c-tg, H-2^{b}) which are transgenic mice of the Class I MHC antigen L^{d}-reactive T cell receptor (TCR) are described in the reference Sha, W.C. *et al.,* Nature, 1988, 335: 271-274. Mice were all treated humanely, maintained in specific-pathogen-free facilities, and fed with standard rodent diet and tap water. Mice at 7 to 9 weeks of age were used. Animal experiments were inspected by the Ethics Committee for Animal Experiments and Recombinant DNA Experiments, Kyushu University, and were performed in accordance with the "Guidelines for Animal Experiments" of the National Institute of Health, U.S.A. "Principles of Laboratory Animal Care" and "Guide for the Care and Use of Laboratory Animals" were also followed.

### Construction of recombinant SeVs

SeVs carrying the EGFP (Jellyfish enhanced green fluorescent protein) gene (SeV-EGFP) or the firefly luciferase gene (SeV-luci) were constructed as described in Kato *et al.* (Kato, A. *et al.,* Genes Cells, 1996, 1: 569-579; Sakai, Y. *et al.,* FEBS Lett., 1999, 456: 221-226). Specifically, an 18 bp spacer sequence 5'-(G)-CGGCCGCAGATCTTCACG-3' (SEQ ID NO: 3) with a *Not* I restriction enzyme site was inserted between the 5'-nontranslated region and the initiation codon of the nucleocapside (N) gene on a vector that harbors a cDNA encoding the SeV genome. This cloned SeV genomic cDNA-comprising vector also contains a self-cleaving ribozyme site derived from the antigenomic strand of the delta hepatitis virus. The entire cDNA encoding EGFP (of SeV-EGFP) or luciferase (of SeV-luci) was amplified by PCR, using primers containing a *Not* I site and sets of E and S signal sequence tags from the new SeV for foreign gene expression, and inserted into the *Not* I site of the above-described cloned genome. The full length of the template SeV genome, including the foreign gene, was arranged to contain multiples of six nucleotides according to the so-called "rule of six" (Kolakofsky, D. *et al.,* J. Virol., 1998, 72: 891-899). The template SeV genome carrying an foreign gene, and plasmids encoding N, phospho(P), and large(L) proteins (pGEM-N, pGEM-P, and pGEM-L, respectively) was complexed with commercially available cationic lipids, and co-transfected with vaccinia virus vT7-3 into CV-1 or LLCMK cells (Fuerst, T.R., Proc. Natl. Acad. Sci. U S A, 1986, 83: 8122-8126). Forty hours later, the cells were disrupted in three cycles of freezing and thawing, and injected into the chorioallantoic cavity of ten-day-old embrionated chicken eggs. The virus was then recovered and the vaccinia virus was eliminated by a second propagation in chicken eggs. Virus titer was determined by hemagglutination assay using chicken erythrocytes (Yonemitsu, Y. & Kaneda, Y., Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999), and the virus was stored at -80°C until use.

### Monoclonal antibodies (mAb)

The biotinylated human CD45 RA (HI100) mAb, allophycocyanin (APC)-conjugated anti-mouse CD3 (145-2C11), APC-conjugated anti-mouse CD8 (53-6.7), and APC-conjugated anti-human CD62L (DREG-56) mAbs, phycoerythrin (PE)-conjugated anti-human CD3 (UCHT1), PE-conjugated anti-human CD4 (RPA-T4), PE-conjugated anti-mouse CD4 (GK1.5), and PE-conjugated anti-mouse CD8 (53.67) mAbs, PE-conjugated strepavidin, as well as peridininchlophyll α protein (perCP)-conjugated streptavidin were purchased from PharMingen (San Diego, CA, USA).

APC-conjugated anti-human CD3 (UCHT1) mAb was purchased from DAKO (Kyoto, Japan). PE-conjugated anti-human CD8 (NU-Ts/c) mAb was purchased from Nichirei (Tokyo, Japan). Anti-2C clonotypic TCR determinant mAb was purified by the present inventors from hybridoma culture supernatants (1B2) (Sha, W.C. *et al.,* Nature, 1988, 335: 271-274) using a HiTrap protein G column (Amersham Pharmacia Bioscience Inc., Buckinghamshire UK), and biotinylated using an EZ-Link™ NHS-LC Biotin (PIERCE Biotechnology Inc., Rockfold, IL, U.S.A.).

For T cell activation, purified anti-mouse-CD3 (145-2C11), mouse-CD28 (37.51), anti-human-CD3 (HIT3a), and -CD28 (CD28.2) mAbs purchased from PharMingen (San Diego, CA, USA) were used.

### Preparation of cells

RPMI 1640 medium (SIGMA, St. Louis, MO, U.S.A.) supplemented with 20 mM N-2-hydroxyethylpiperazine-N'-2-ethanesulfonic acid (HEPES), 0.2% sodium bicarbonate, 50 µM 2-mercaptoethanol (2-ME), 10 µg/ml gentamicine sodium, and heat-inactivated 10% fetal bovine serum (FBS) (ICN Biomedicals, Inc., Arora, OH, U.S.A.) was used as a complete medium.

For preparation of mouse lymphocytes, spleen and lymph nodes were collected and kept on ice in complete medium. The spleen and lymph nodes were disrupted in the medium by pressing these tissues between two glass slides. The cell suspension thus prepared was filtered through a stainless mesh, and washed twice with the medium. Erythrocytes were lysed using ammonium chloride potassium carbonate lysis buffer. Human peripheral blood lymphocytes (PBL) were separated using Ficoll Paque™ PLUS (Pharmacia Biotech Inc., Wikstroms, Sweden) from blood provided by healthy donors. Viable nucleated cells were counted using a standard trypan blue (dye) exclusion assay system.

### Establishment of mouse and human T cell lines

To prepare alloreactive T cell lines, B6 or 2C-tg mouse lymphocytes (5x 10⁷) were co-cultured with 30 Gy- irradiated (¹³⁷Cs; Gammacell 40, Atomic Energy of Canada Limited, Ottawa, Canada) Balb/c lymphocytes (5x 10⁷) in RPMI 1640 complete medium (total volume: 10 ml) in a 50-ml flask (35-3014; FALCON, Beckton Dickinson Bioscience, Inc., Franklin Lake, NJ, U.S.A.) for six days. Following the addition of human IL-2 (10 ng/ml) (Immuno-Biological Laboratories Co., Ltd, Fujioka, Japan), activated alloreactive T cells were stimulated with the irradiated Balb/c lymphocytes once a week. Since the T cell line obtained from B6 using this method consisted mostly CD8 T cells, in order to obtain CD4 T cell lines, CD8-depleted B6 lymphocytes were stimulated with the irradiated lymphocytes. To prepare CD8-depleted lymphocytes, newly isolated lymphocytes were incubated with an anti-mouse CD8 mAb (Lyt-2.2: Meiji, Tokyo, Japan) for 30 min at 4°C, and then with a Low-Tox™-M Rabbit completent (Cedarlane, Ontario, Canada) for 50 min at 37°C. B6 or 2C-tg alloantigen-activated T cells stimulated three times or more were used as the murine T cell line.

To prepare human T cell lines, periphertal blood lymphocytes (5x 10⁶) provided by a healthy donor were co-cultured with 30 Gy-irradiated peripheral blood lymphocytes (5x 10⁶) from a different healthy donor, in the presence of human IL-2 (10 ng/ml) in RPMI-1640 infection medium (1 ml) for seven days. After that, the lymphocytes were re-stimulated with the irradiated lymphocytes at least twice a week and were used as a T cell line.

### Flow cytometry analysis

Collected murine cells were centrifuged, and incubated with the culture supernatant (50 µl) of anti-mouse CD16/32 mAb-producing hybridoma (2.4G2; American type culture collection, Manassas, VA, U.S.A) for 30 min at 4°C. This step was omitted for the human lymphocytes. Cells were washed in complete medium, incubated with various combinations of mAbs for 30 min at 4°C, and again washed twice with the complete medium. The biotinylated mAb was detected with PE-streptavidin or PerCP streptavidin. Labelled cells were analyzed using a FACS Caliber with the CellQuest program (Becton Dickinson, San Jose, CA, USA), and the FLOWJO program (TREE STAR, Inc., San Carlos, CA, USA). In order to detect and eliminate dead cells, 125 ng of propidium iodide (PI) was added to the cell suspension (250 µl) right before cytometer application. This step was omitted in the separation of naive T cells from human activated/memory T cells. EGFP was detected with fluorescence 1. In both murine and human studies, the CD3⁺ CD4⁺ PI⁻ cell population represents viable CD4 T cells and the CD3⁺ CD8⁺ PI⁻ cell population represents viable CD8 T cells. The CD8⁺ 1B2⁺ PI⁻ cells, which are transgenic clonotypic T cells, represent viable 2c T cells. Naive human T cells were determined to be those gated (sorted) as CD62L⁺ CD45RA⁺ CD3⁺ cells, and activated/memory human T cells as those excluded from CD3⁺ cells (Picker, L.J. *et al.,* J. Immunol., 1993, 150: 1105-1121; Ostrowski, M.A. *et al.,* J. Virol., 1999, 73: 6430-6435).

### Gene delivery into murine or human T cells by SeV

To assess the gene transduction efficiency into activated or naive T cells, 200 µl of murine lymphocyte suspension (2x 10⁶/ml) was cultured with SeV-EGFP (2.5x 10⁷ plaque forming unit (PFU)) for two days, in a 96-well flat-bottomed plate (3860-096; IWAKI, Tokyo, Japan) which was either non-coated or coated with the anti-mouse CD3 mAb (15 µg/ml) and the anti-mouse CD28 mAb (20 µg/ml). As for the human lymphocytes, 200 µl of the human peripheral blood lymphocyte (PBL) suspension (4x 10⁶/ml) or the human T cell line (4x 10⁶ /ml) was cultured with SeV-EGFP (2.5x 10⁷ PFU) for two days, in a 96-well flat-bottomed plate either non-coated or coated with the anti-human CD3 mAb (10 µg/ml) and the anti-human CD28 mAb (10 µg/ml). Assessment of gene transduction efficiency in alloactivated T cell lines was performed by co-culturing 100 µl of the B6 or 2C-tg mouse T cell line (2x 10⁶ /ml) with 100 µl of 30 Gy-irradiated B6 or Balb/c mouse lymphocytes (1x 10⁷ /ml) in the presence of SeV-EGFP (2.5x 10⁷ PFU) for two days. Alternatively, this could be done by co-culturing 200 µl of the B6 or 2C-tg mouse T cell line (2x 10⁶/ml) with SeV-EGFP (2.5x 10⁷ PFU) in a 96-well flat-bottomed plate coated with antibodies (anti-CD3 antibody and anti-CD28 antibody) for two days. To assess the gene transduction efficiency under alloantigen-specific activation of naive T cells, fresh lymphocytes isolated from 2C-tg mouse were used. In an alternative experiment, 2.5 ml of a T cell line (2x 10⁶ /ml) from 2C-tg mouse was stimulated for six days with 2.5 ml of 30 Gy-irradiated lymphocytes (1x 10⁷ /ml) from B6 or Balb/c mouse (half of the medium was replaced with fresh medium every three or four days) in the presence of SeV-EGFP (6x 10⁸ PFU). The transduced T cells were washed with fresh medium, and re-stimulated with the irradiated B6 or Balb/c lymphocytes in the absence of SeV for six or seven days (restimulation was performed every six or seven days). To assess the effects of bystander activation, a mixed suspension of 50 µl 2C naive lymphocytes (1x 10⁷ /ml) and 50 µl B6 naive lymphocytes (1x 10⁷/ml) was stimulated for two days with 100 µl of irradiated Balb/c, B6, or C3H lymphocytes (1x 10⁷/ml), in the presence of SeV-EGFP (2.5x 10⁷ PFU).

Each sample was cultured in triplicates at 37°C in humidified air containing 5% CO₂. The EGFP expression level reached maximum 48 h after SeV infection. The appropriate concentration of activating mAb or optimal dose of SeV was determined by titration experiments. The lowest SeV dose for transducing the maximum EGFP into T cells was a multiplicity of infection (MOI) of 12.5, and SeV MOI over 500 had cytopathic effects on T cells.

### Assessment of SeV entry into naive or activated T cells

B6 lymphocytes (4x 10⁶/ml) were incubated in the presence or absence of SeV-luci (5x 10⁸ PFU/ml) inactivated with 2000 mj UV (UV crosslinker; Pharmacia Biotech Inc., San Francisco, CA, USA) at a 1:1 ratio for 30 min at 37°C. The cells were washed thoroughly with a complete medium, and incubated with SeV-EGFP (2.5x 10⁸ PFU/ml) at a 1:1 ratio for 30 min at 37°C. After three washes, 200 µl of the cell suspension (2x 10⁶/ml) was cultured for two days in a 96-well flat-bottomed plate coated with the anti-mouse CD3 mAb (15 µg/ml) and anti-mouse CD28 mAb (20 µg/ml). Contaminant SeV present in the final washing medium had almost no ability to transduce EGFP into T cells that had not been pre-treated in the activating wells, and therefore this washing process was confirmed to be sufficient. For the positive control, prepared naive cells were cultured with SeV-EGFP (2.5x 10⁷ PFU) in activating wells for two days. To examine the release of attached SeV from naive T cells, 10 ml of B6 lymphocytes (2x 10⁶ /ml) were incubated in the presence or absence of SeV-EGFP (2x 10⁹ PFU) for 30 min at 4°C, washed thoroughly with the complete medium, and then incubated in the absence SeV for 0, 15, 30, 45, or 90 min at 37°C. After three washes, cells were cultured in activating wells at 37°C for two days. For the positive control, prepared naive cells were cultured with SeV-EGFP (2.5x 10⁷ PFU).

### [Example 1] Recombinant SeV transduces EGFP into activated T cells with a high efficiency

Whether Sendai virus vectors can transduce EGFP gene into T cells was examined. First, murine lymphocytes were cultured with SeV-EGFP (MOI=62.5) for 48 h. While the ratio of EGFP-positive cells in unstimulated murine CD3⁺ CD4⁺ or CD3⁺ CD8⁺ T cells (also referred to as CD4 T cells or CD8 T cells) was low (0.5 to 1.5% and 0.8 to 2.0%, respectively), CD3⁺ CD4⁺ or CD3⁺ CD8⁺ T cells non-specifically activated with the immobilized anti-CD3 antibody and anti-CD28 antibody expressed EGFP at high levels, and the ratio of EGFP-positive T cells dramatically increased (65 to 85% and 70 to 92%, respectively) (Fig. 1). In both cases of CD4 and CD8 T cells, the ratio of EGFP-positive cells increased in a SeV dose-dependent manner and nearly reached a plateau level at an MOI of 12.5.

Next, to examine whether it is possible to transduce genes into antigen-acitvated T cell lines, an alloantigen was used as a T cell-stimulating antigen, to which naive lymphocytes can respond and proliferate in primary cultures without *in vivo* immunization. The T cell line generated by co-culturing unmodified lymphocytes from C57BL/6 and irradiated lymphocytes from Balb/c consisted mostly CD8 T cells. A CD4 T cell line was then obtained by co-culturing CD8 T cell-depleted lymphocytes with the irradiated stimulating lymphocytes. Alternatively, T cells from 2C-tg mouse (2C T cells) were used, which had a large quantity of naive T cell clones expressing L^{d}-specific TCR, and thereby enabled the observation of gene transduction in the antigen-specific response of primary T cells. As expected, T cells stimulated with an alloantigen were efficiently transduced with EGFP by SeV in the presence of irradiated allogenic lymphocytes (Fig. 2). Further, even in the absence of stimulating allogenic lymphocytes, activated T cells were efficiently transduced with the gene (Fig. 2). Although this effect was common in both CD4 and CD8 T cells, the EGFP expression level and EGFP-positive cell ratio tend to be slightly lower in the CD4 T cell line than CD8 T cell line. These findings suggest that SeV is able to transduce a target gene into T cells that are in an activated state.

T cells from 2C-tg mice were used to confirm the findings in antigen-specific T cell response. These 2C-tg murine T cells respond specifically to L^{d} and can be separated as a CD8⁺ 1B2⁺ population from bulk lymphocytes even in their naive state. EGFP was highly expressed in naive 2C T cells under the sole presence of allogenic Balb/c stimulating cells, but was rarely expressed in the presence of syngenic B6 stimulator cells. However, SeV efficiently transduced EGFP into activated 2C T cells that have been stimulated three times or more with Balb/c stimulator cells, in the presence of either B6 or Balb/c stimulator cells (Fig. 3). In addition, simple incubation of T cell lines with SeV for only 30 min at 37°C was sufficient for the maximum expression of EGFP (data not shown).

Further, since non-antigen specific T cells could be activated *in vitro* by the bystander effect of a strong alloresponse, the following experiment was performed to verify whether this powerful SeV gene transduction is limited to antigen-specific activated T cells. Naive 2C T cells were used as antigen-specific T cells that could respond to Balb/c stimulators but not to C3H stimulators. Rresponders (responding cells) comprising mixtures of C57BL/6 and naive 2C T cells were cocultured with or without Balb/c, C57BL/6, or C3H stimulator cells, followed by addition of SeV-EGFP (2.5x 10⁷ PFU) into the culture wells. With this culture system, whether EGFP gene was transduced into 2C T cells by SeV-EGFP could be examined when T cells from C57BL/6 mice were responding strongly to the C3H stimulators in the mixed lymphocyte culture. When C3H stimulator cells were used, even though C57BL/6 T cells responded to the C3H stimulators, EGFP was not transduced into 2C T cells. This same result was observed when no stimulator cells were used, and when C57BL/6 stimulator cells were used (Fig. 4). In contrast, when Balb/c stimulators was used, 2C T cells expressed EGFP at high levels (Fig. 4). Therefore, this gene transduction via SeV was limited to T cells activated with specific antigens.

### [Example 2] Duration of transgne expression by SeV in activated T cells

Next, *in vitro* maintenance of the transduced gene was examined. Activated 2C T cells were cocultured with the Sendai virus, and the transduced T cells were maintained *in vitro* with either Balb/c stimulators or C57BL/6 stimulators. EGFP expression level rapidly decreased following a peak expression at 48 h after the infection, but was maintained for at least 20 days (Fig. 5 and data not shown). EGFP expression level was not elevated, even with antigen re-stimulation using the Balb/c stimulators. These findings were observed also in the allospecific activated T cell lines (data not shown).

### [Example 3] Gene delivery into activated human T cells and T cell lines

Freshly isolated human PBL from healthy donors were cultured with 2.5x 10⁷ PFU of SeV-EGFP expression vector (MOI=30) for 48 h. In contrast to murine T cells, although EGFP expression intensities were relatively low in unstimulated human CD3⁺ CD4⁺ and CD3⁺ CD8⁺T cells, relatively high EGFP positive ratios were obtained (mean ratio = 23.1% in the range of 15 to 45%, and mean ratio = 34.0% in the range of 18 to 50%, respectively) (Fig. 6).

The present inventors hypothesized that the activated/memory T cell populations might be higher in human PBL than in mouse lymphoid tissues that had been maintained under specific-pathogen-free conditions. Naive T cells (CD45RA⁺ CD62L⁺) were separated from activated/memory T cells, and the EGFP expression of each T cell population was analzyed. As expected, the EGFP-positive activated/memory T cells had an exceptionally higher ratio of EGFP-positive cells than naive T cells which had a ratio below 4% (Fig. 7). Mouse studies showed that even in the absence of antigen stimulation, EGFP was efficiently transduced into T cells that had been preactivated with an antigen. Thus it was thought the activated T cells among the activated/memory phenotypic T cells were expressing EGFP. In constrast, the CD3⁺ CD4⁺ or CD3⁺ CD8⁺ T cells, which had been stimulated using the immobilized anti-CD3 antibody and anti-CD28 antibody, expressed EGFP intensely and had a high ratio of EGFP-positive cells (in the range of 30 to 69% and 50 to 70%, respectively) (Fig. 6).

In the human alloantigen-stimulated T cell line, both CD4 and CD8 T cells showed exceptionally efficient gene transduction, 97% and 98% respectively, in the presence of immobilized antibodies (Fig. 8). With this T cell line, simple incubation for only 30 min at 37°C was sufficient for the maximum EGFP expression.

### [Example 4] Post-attachment entry of the SeV vector occurs in activated T cells but not in naive T cells

Possible mechanisms of the activated T cell-specific gene transduction mediated by SeV were examined. The following factors are likely to affect gene transduction efficiency: (i) SeV specific receptors (Markwell, M.A. and Paulson, J.C., Proc. Natl. Acad. Sci. U S A, 1980, 77: 5693-5697), (ii) putative co-receptors required for fusion (Kumar, M. *et al.,* J. Virol., 1997, 71: 6398-6406; Eguchi, A. *et al.,* J. Biol. Chem., 2000, 275: 17549-17555), and (iii) induction of signal transduction by T cell activation, which can affect SeV transcription after entry (Collins, P.L. *et al.,* Parainfluenza viruses. In: Fields BN, Knipe DM, Howley PM, (eds). Fields of virology. Vol. 2. Lippincott - Raven Publishers: Philadelphia, 1996: 1205-1241). Murine T cells were used in the investigation of these factors. SeV-mediated gene transduction was efficient in activated murine T lymphocytes, but was almost not seen with freshly isolated murine T lymphocytes. Therefore, in experiments using murine T cells, the procedure of separating naive T cells from activated/memory T cells was omitted.

In order to interfere with the T cell specific receptor and release sialic acid residues using the HN neuraminidase activity of SeV, freshly isolated lymphocytes were first incubated in the presence (pre-treated naive T cells) or absence (naive T cell without pretreatments) of UV-inactivated SeV-luci for 60 min at 37°C. These T cells were then incubated with SeV-EGFP (MOI=62.5) for 30 min at 37°C, and stimulated with the immobilized anti-CD3 and anti-CD28 antibodies. Two days later, EGFP expression levels were examined. In SeV-EGFP-pretreated naive T cells, the ratios of EGFP-positive cells in CD4 and CD28 T cells under continuous activation became 35% and 50%, respectively (Fig. 9). However, when naive T cells were incubated with UV-inactivated SeV-luci, transduction of the EGFP gene into activated T cells was inhibited. Most of the T cells that had been incubated with SeV-EGFP during the activation culture period expressed EGFP even if they were preincubated with inactivated SeV-luci, therefore the absence of EGFP expression in pretreated T cells was not due to decreased T cell viability (Fig. 9). Instead, this was thought to result from the recovery of SeV-specific receptors during the subsequent incubation period. Further, since the final washing medium transduced little EGFP into unmodified activated T cells (data not shown), the possibility of an insufficient washing process was eliminated. These findings suggest that SeV uses a specific receptor on T cells for gene transduction.

In a preliminary experiment that determined the T cell incubation time with SeV-EGFP, it was observed that the ratio of EGFP-positive T cells decreased with extended incubation time. From this observation, the present inventors hypothesized that SeV adheres to but does not fuse with naive T cells presumably because of co-receptor deficiency, which leads to the dissociation of SeV from naive T cells as a result of its own HN neuraminidase activity at the incubation temperature of 37°C, which is optimal for the enzyme's activity. Then, freshly isolated lymphocytes were incubated with SeV-EGFP (MOI=100) at 4°C, where neuraminidase hardly functions but SeV can still adhere to the sialic acid residues, followed by incubation in a fresh medium at 37°C for the indicated periods (Fig. 10). These T cells were then washed three times and stimulated with immobilized anti-CD3 and anti-CD28 antibodies, and their GFP expression levels were examined two days later. In this experiment, all T cells were equally activated in a way that data were not affected by factors such as cellular kinase activities associated with SeV gene expression, but only by factors associated with SeV entry. When T cells were incubated once at 4°C and then activated, ratios of EGFP-positive CD4 and CD28 T cells were 50% and 70% respectively. In contrast, a subsequent incubation at 37°C following 4°C, ratios of EGFP-positive cells decreased with time (Fig. 10). Ratios of EGFP-positive cells reached their minimum after 90 minutes of incubation at 37°C, and were equivalent to co-culturing T cells that had not been pretreated with SeV mixed in the culture medium after the final wash in activating wells. Positive control T cells incubated with SeV-EGFP during the culture period for activation were all transduced efficiently with the gene. Further, when activated T cells were used, the ratio of EGFP-positive T cells after only 30 min of incubation at 37°C was as high as that of the positive control (data not shown). Since SeV which has once entered naive T cells is not likely to be released therefrom, these phenomena can be interpreted as the ability of SeV to enter activated T cells, and to adhere to naive T cells but not fuse with them. It is highly possible that the vector particle causes internalization specific to activated T lymphocytes but not to naive T lymphocytes.

### Industrial Applicability

The present invention enables efficient gene transduction into T cells. Since gene transduction into T cells is important for treating various diseases associated with the immune system, the present invention is expected to be applied in immunological modification strategies using T cell-directed gene delivery in these diseases.

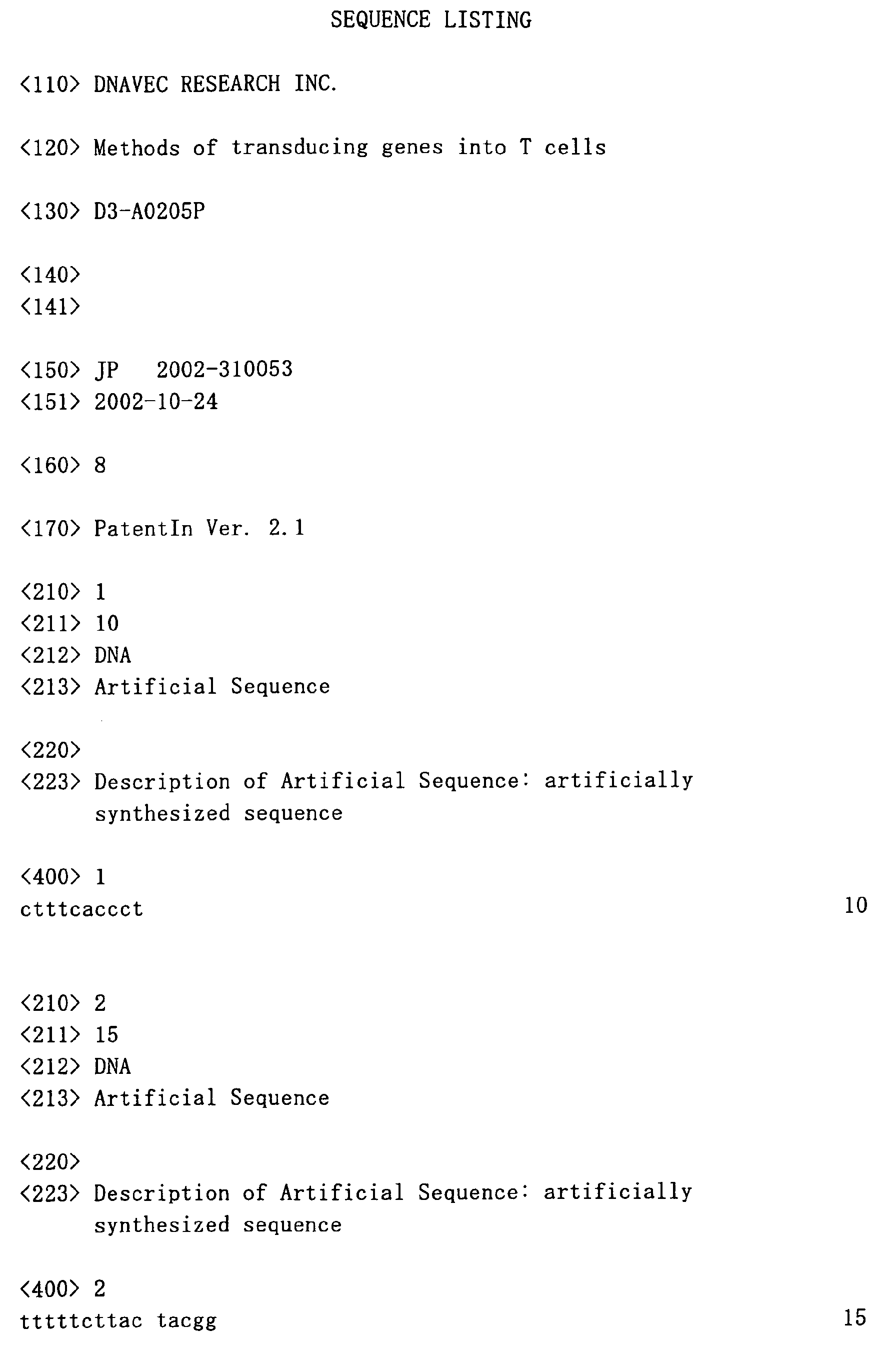

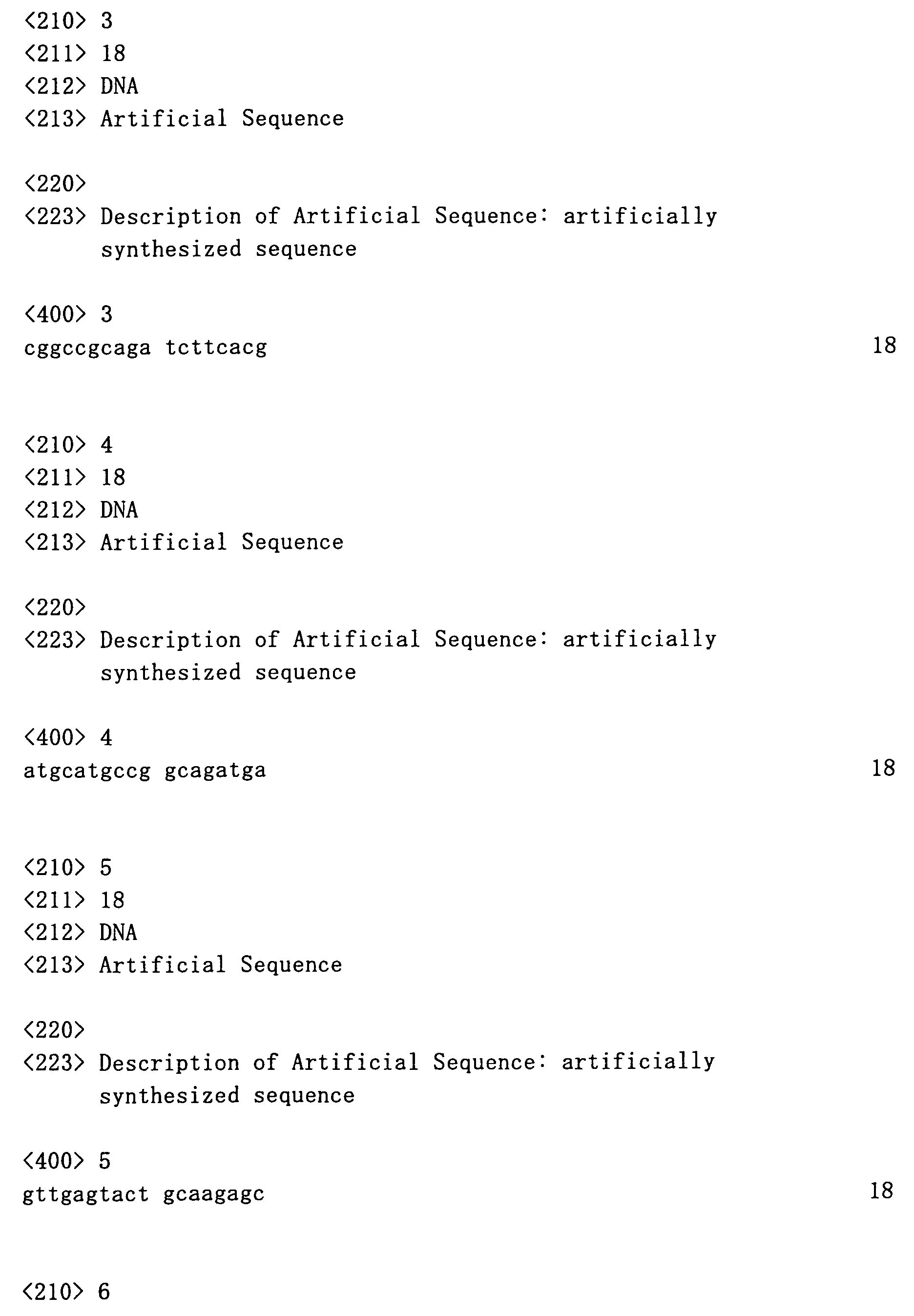

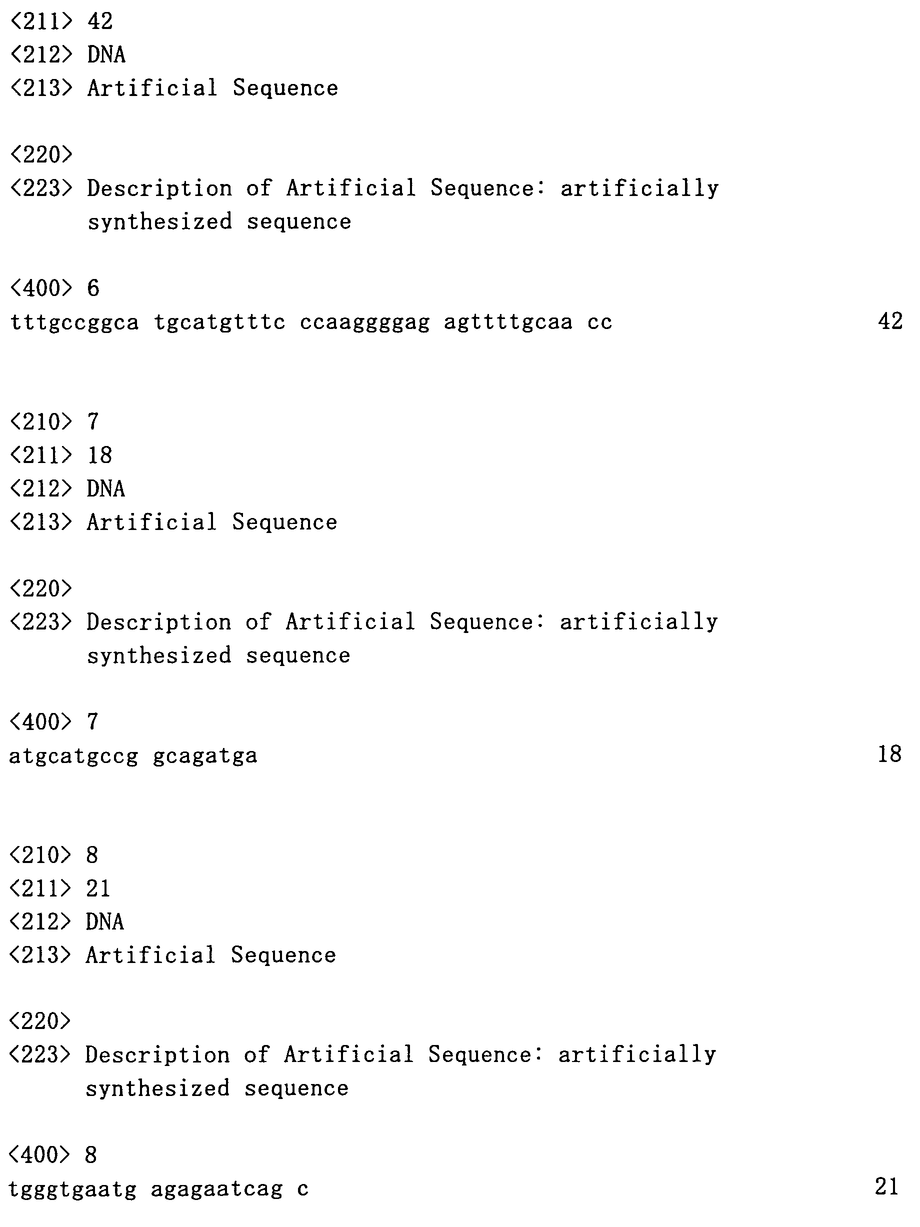

## Claims

1. A method for transducing a gene into T cells, wherein said method comprises the step of contacting a paramyxovirus vector carrying the gene with activated T cells.

2. The method according to claim 1, wherein the paramyxovirus vector is a Sendai virus vector.

3. A method of preparing T cells transduced with a foreign gene, wherein said method comprises the step of contacting a paramyxovirus vector carrying said gene with activated T cells.

4. The method according to claim 3, wherein the paramyxovirus vector is a Sendai virus vector.

5. A T cell transduced with a foreign gene prepared by the method according to claim 3 or 4.

6. A paramyxovirus vector to be used in gene transduction into activated T cells.

7. The vector according to claim 6, wherein the paramyxovirus vector is a Sendai virus vector.
